# EUROPEAN PATENT APPLICATION

(11) **EP 3 991 728 A1**
(43) Date of publication of application: **04.05.2022**
(21) Application number: 20831464.1
(22) Date of filing: 24.06.2020
(51) Int. Cl.: A61K 31/353, A23L 33/105, A61P 1/16, A61P 43/00

(54) **HEPATIC FIBROSIS-INHIBITING AGENT AND BROWN FAT CELL-ACTIVATING AGENT CONTAINING TAXIFOLIN**

(30) Priority: 25.06.2019 JP 2019117229
(71) Applicant: National Hospital Organization, Tokyo 152-8621 (JP); National Cerebral and Cardiovascular Center, Suita-shi, Osaka 564-8565 (JP); Advanced Innovation Development Co. Ltd., Tokyo 101-0052 (JP); Foundation for Biomedical Research and Innovation at Kobe, Kobe-shi, Hyogo 650-0047 (JP)
(72) Inventor: ASAHARA, Noriko, Kyoto-shi, Kyoto 612-8555 (JP); INOUE, Takayuki, Kyoto-shi, Kyoto 612-8555 (JP); TANAKA, Masashi, Kyoto-shi, Kyoto 612-8555 (JP); IHARA, Masafumi, Kishibe-Shimmachi, Suita-shi, Osaka 564-8565 (JP); SAITO, Satoshi, Kishibe-Shimmachi, Suita-shi, Osaka 564-8565 (JP)
(74) Representative: J A Kemp LLP
(86) International application number: PCT/JP2020/025740
(87) International publication number: WO 2020/262703

(57) **Abstract**

The present invention aims to provide a novel liver fibrosis inhibiting agent or brown adipocyte activating agent, and a prophylactic and/or therapeutic agent for nonalcoholic steatohepatitis which contains the inhibiting agent or activating agent as an active ingredient. The present invention relates to a liver fibrosis inhibiting agent and a brown adipocyte activating agent, each containing taxifolin as an active ingredient, and a prophylactic and/or therapeutic agent for nonalcoholic steatohepatitis, containing taxifolin as an active ingredient.

## Description

### [Technical Field]

The present invention relates to a liver fibrosis inhibiting agent and a brown adipocyte activating agent each containing taxifolin as an active ingredient. In addition, the present invention also relates to use of a taxifolin-containing liver fibrosis inhibiting agent for the prophylaxis and/or treatment of nonalcoholic steatohepatitis.

### [Background Art]

Obesity is increasing in Japan due to westernization of eating habits, lack of exercise, and the like, and those with diabetes or metabolic syndrome, including people at risk thereof, amount to 22 million and 20 million, respectively. Furthermore, in recent years, with the increase in the obese population, the number of people with nonalcoholic fatty liver diseases (hereinafter referred to as "NAFLD") such as nonalcoholic steatohepatitis (hereinafter referred to as "NASH") and the like has increased rapidly to about 30% of medical examinations, estimated at 15 - 23 million people. According to the latest data, it is assumed that about 25% of them progress to NASH, and when NASH fibrosis progresses, about 25% develop cirrhosis, and about 25% of them develop hepatocellular cancer in 10 years. However, the mechanism by which fatty liver progresses to NASH is still unknown, and since definitive diagnosis of NASH requires invasive liver biopsy, large-scale research is difficult, and a clear progression factor or biomarker has not been established (non-patent document 1). Furthermore, the treatment method at present is basically the improvement of lifestyle such as diet•exercise therapy. As for the drug therapy, there are few reports with a high evidence level, and a standard treatment method has not been established. Now that viral hepatitis can be treated, measures for the prophylaxis and treatment of NASH are urgent issues.

Obesity is a state in which fat is accumulated and enlarged in white adipocytes, and white adipocytes differentiate to increase the number thereof. In contrast, while brown adipocyte stores fat in the same manner as white adipocyte, the action of uncoupling protein 1 (hereinafter referred to as "UCP1") present in mitochondria converts fat into heat without being subjected to ATP synthesis in the electron transport chain. In the living body, it is considered that systemic energy metabolism and body fat regulation are conducted by energy storage by white adipocytes, energy supply via the ATP synthesis system, and energy consumption by heat production by brown adipocytes. In recent studies, it has been found that brown adipocyte includes two types: "classical type" whose cell fate has already been determined at the initial stage of embryonic development, and "inducible type" which is induced to differentiate from white adipocytes by various environmental factors and the like in adults (non-patent document 2). In humans, brown adipocytes of "classical type" are present in small amounts in limited areas such as around the scapula, behind the neck, around the kidneys and the like, and decrease with age. It has been reported that white fat progenitor cells present in white adipose tissue of adults differentiate into brown adipocytes of "inducible type" due to environmental factors such as long-term cold exposure and the like. Therefore, it is possible to prevent or improve metabolic syndrome (e.g., obesity, diabetes, dyslipidemia, hypertension, etc.) and the like by promoting the differentiation of white fat progenitor cells into brown adipocytes, and promoting energy consumption by activating brown adipocytes to suppress the accumulation of excess fat in the body. In general, chronic inflammation derived from metabolic syndrome or the like induces stroma fibrosis and eventually leads to organ dysfunction. This is no exception to adipose tissue. Recently, it has been reported that PRDM16, which is a transcription factor that acts on the differentiation of brown adipocyte and maintenance of the function thereof, suppresses fibrosis of adipose tissue regardless of heat production (UCP1-independently) (non-patent document 3).

In recent years, plant-derived preparations are attracting attention as the safest drugs. Among them, flavonoids, which are active ingredients in plant-derived preparations, are known for their unique anti-oxidation activity and biological effects over the wide range (immune enhancing action, antitumor action, cardiac protection action, radiation disorder protection action, anti-aging action, antiplatelet action, antiallergic action, antivirus action, etc.) (non-patent documents 4 - 6). Particularly, various plant-derived preparations are produced in Russia, and more than 200 kinds of raw plant materials are used for medical purposes. About 40% of the types of drugs produced in Russia are plant-derived preparations and flavonoid is contained in most of them.

Recently, diquvertin which is a bioflavonoid complex separated from the xylem of dahurian larch or Larix sibirica was added to the list of flavonoid-containing drugs. Taxifolin accounts for the most part (not less than 90%) of the active ingredient of "diquvertin" (non-patent document 7). Taxifolin has been reported to have anti-oxidation property and capillary protection property (non-patent document 8), and has been approved by the Ministry of Health of the Russian Federation as a pharmaceutical product showing capillary protection and anti-oxidation property. It is also approved as a functional food and as a food additive that extends the best-by date of foods. Furthermore, taxifolin has recently been confirmed to be safe for intake (non-patent document 9), as well as has an anti-oxidation action in acute pancreatitis model mouse (non-patent document 10), blood glucose-improving effects in type 1 diabetes model rat (non-patent document 11), and dementia-improving effects such as suppression of amyloid β coagulation, improvement of brain blood flow, improvement of spatial recognition function of mouse in the water maze test, and the like in Alzheimer's disease model mouse (patent document 1, non-patent document 12).

However, there are no reports on the liver fibrosis suppressive action or brown adipocyte activation action of taxifolin, and there are no reports on the use of taxifolin as a prophylactic and/or therapeutic agent for NASH accompanied by fibrosis.

### [Document List]

### [Patent document]

Patent document 1: WO 2017/199755

### [Non-patent documents]

Non-patent document 1: NAFLD/NASH guideline for the Clinical Practice of 2014, edited by the Japanese Society of Gastroenterology, Nankodo Co., Ltd.
Non-patent document 2: Cell Metabolism, 2010, 11: 257-262
Non-patent document 3: Cell Metabolism, 2018, 27: 180-194. e6
Non-patent document 4: Pharmacol. Rev., 2000, 52(4): 673-751
Non-patent document 5: Fitoterapia, 1991, 62(5): 371-389
Non-patent document 6: Drug Metabol. Drug Interact., 2000, 17(1-4): 291-310
Non-patent document 7: 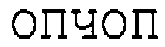 . 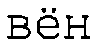 , 1998, 19
Non-patent document 8: 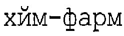 . 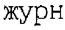 , 1995, 29, 9, 61-64
Non-patent document 9: Int. J. Toxicol., 2015, 34(2), 162-181
Non-patent document 10: J. Ethnopharmacol., 2018, 224, 261-272
Non-patent document 11: J. Cell. Biochem., 2019, 120(1), 425-438
Non-patent document 12: Acta Neuropathol. Commun., 2017, 5(1), 26, doi:10.1186/s40478-017-0429-5

### [Summary of Invention]

### [Problems to be solved by the Invention]

Under such background, the development of a novel liver fibrosis inhibiting agent targeting NASH for which a prophylactic method or a treatment method has not yet been established, particularly, NASH accompanied by liver fibrosis that can progress to cirrhosis or hepatocellular cancer, and creation of a prophylactic agent and/or a therapeutic agent for NASH resulting therefrom, as well as the development of a brown adipocyte activating agent that can effectively promote energy consumption in the body and also suppress fibrosis of adipose tissue have been desired.

The present invention aims to provide a novel liver fibrosis inhibiting agent that suppresses progress of fibrosis in NASH. The present invention also aims to provide a novel brown adipocyte activating agent. Another object of the present invention is to provide a pharmaceutical composition or food composition effective for the prophylaxis and/or treatment (or improvement) of chronic hepatic diseases accompanied by liver fibrosis (hepatitis C, hepatitis B, non-B or non-C type hepatitis such as NASH and the like, autoimmune hepatitis, etc.), particularly, NASH accompanied by liver fibrosis.

### [Means of Solving the Problems]

The present inventors have conducted intensive studies under such circumstances and found for the first time that taxifolin or a salt thereof exhibits a superior liver fibrosis suppressive action and a superior brown adipocyte activation action, and may provide a useful and safe pharmaceutical composition or food composition for the prophylaxis and/or treatment of NASH accompanied by liver fibrosis, for which there was no effective prophylactic method or treatment method, and completed the present invention.

Accordingly, the present invention provides the following.
[1] A liver fibrosis inhibiting agent comprising taxifolin as an active ingredient.
[2] The liver fibrosis inhibiting agent of the above-mentioned [1], wherein the liver fibrosis is caused by hepatitis virus infection, nonalcoholic steatohepatitis, or autoimmune hepatitis.
[3] The liver fibrosis inhibiting agent of the above-mentioned [1], wherein the liver fibrosis is caused by nonalcoholic steatohepatitis.
[4] A brown adipocyte activating agent comprising taxifolin as an active ingredient.
[5] The brown adipocyte activating agent of the above-mentioned [4] for suppressing liver fibrosis.
[6] The brown adipocyte activating agent of the above-mentioned [5], wherein the liver fibrosis is caused by hepatitis virus infection, nonalcoholic steatohepatitis, or autoimmune hepatitis.
[7] The brown adipocyte activating agent of the above-mentioned [5], wherein the liver fibrosis is caused by nonalcoholic steatohepatitis.
[8] A prophylactic and/or therapeutic agent for nonalcoholic steatohepatitis accompanied by liver fibrosis, comprising the agent of any of the above-mentioned [1] to [7] as an active ingredient.
[9] The brown adipocyte activating agent of the above-mentioned [5] for promoting energy consumption.
[10] The brown adipocyte activating agent of the above-mentioned [5] for promoting expression of UCP1.
[11] The agent of any of the above-mentioned [1] to [10], wherein the agent is prepared in the form of a tablet, pill, capsule, powder, granule, fine granules, troche, or liquid.
[12] A prophylactic and/or therapeutic agent for nonalcoholic steatohepatitis accompanied by liver fibrosis, comprising a prophylactically and/or therapeutically effective amount of taxifolin or a salt thereof as an active ingredient.
[13] A food composition for the prophylaxis or improvement of nonalcoholic steatohepatitis accompanied by liver fibrosis, comprising an effective amount of taxifolin or a salt thereof as an active ingredient.
[14] The food composition of the above-mentioned [13], comprising an indication that the composition is for "reducing a risk of liver fibrosis" and/or "promoting energy metabolism".
[15] The food composition of the above-mentioned [13] or [14], which is prepared as a supplement, a functional food, a health food, a food for special dietary uses, a health-promoting food, a food for specified health uses, or a food with nutrient function claims.
[16] The food composition of any of the above-mentioned [13] to [15], which is prepared in the form of tablet, pill, capsule, powder, granule, fine granule, troche, or liquid.
[17] A method for preventing and/or treating nonalcoholic steatohepatitis accompanied by liver fibrosis, comprising orally administering a prophylactically and/or therapeutically effective amount of taxifolin or a salt thereof to a subject.
[18] Taxifolin or a salt thereof for use in the prophylaxis and/or treatment of nonalcoholic steatohepatitis accompanied by liver fibrosis.
[19] A pharmaceutical composition comprising taxifolin for use in the prophylaxis and/or treatment of nonalcoholic steatohepatitis accompanied by liver fibrosis.
[20] A food composition comprising taxifolin for use in the prophylaxis and/or treatment of liver fibrosis.
[21] Use of taxifolin or a salt thereof in the production of a medicament for use in the prophylaxis and/or treatment of nonalcoholic steatohepatitis accompanied by liver fibrosis.

### [Effect of the Invention]

According to the present invention, a superior liver fibrosis inhibiting agent characteristically containing taxifolin, which is a safe plant-derived component, or a salt thereof as an active ingredient can be provided. Using this, the progression of fibrosis in NASH can be suppressed. According to the present invention, moreover, a superior brown adipocyte activating agent containing taxifolin or a salt thereof as an active ingredient can be provided. Using this, it is possible to promote energy consumption, enhance the expression of UCP1, and effectively suppress the fibrosis of adipose tissue. As a result, metabolic syndrome and the like can be prevented or improved. Furthermore, according to the present invention, a medicament or food composition useful for the prophylaxis and/or treatment (or improvement) of liver fibrosis induced by various causes (hepatitis virus infection, NASH, autoimmune hepatitis, etc.), by administering a prophylactically and/or therapeutically effective amount of taxifolin or a salt thereof, which is a liver fibrosis inhibiting agent and/or a brown adipocyte activating agent, to a subject can be provided. Among others, according to the present invention, a novel and effective prophylactic agent for NASH accompanied by fibrosis, for which there was no effective prophylactic or treatment method, can be provided. It is also possible to potentiate the prophylactic and/or treatment effects of taxifolin or a salt thereof in the present invention by combining with dietary therapy, exercise therapy, drug therapy (combination therapy with other drugs), iron removal therapy, and/or surgical treatment (debulking operation, liver transplantation).

### [Brief Description of the Drawings]

Fig. 1A is a graph showing changes in the body weight of mice in a food ingestion experiment, and Fig. 1B shows photographs of the appearance of the mice in respective groups on the last day of the food ingestion experiment.
Fig. 2 is a graph showing changes in the amount of food intake by the mice in the food ingestion experiment period.
Fig. 3A is a graph showing changes in the fasting blood glucose, Fig. 3B is a graph showing changes in the blood glucose level at 30 min, 60 min, and 120 min with the intraperitoneal glucose tolerance test start time as 0 min, and Fig. 3C is a graph showing the postprandial blood glucose area under the curve of the mice in respective groups.
Fig. 4A is a graph showing changes in the rectal temperature of mouse at 8 weeks and 12 weeks after the start of the food ingestion experiment, and Fig. 4B is a graph showing UCP1 gene expression in the mice in respective groups at 12 weeks after the start of the food ingestion experiment.
Fig. 5 shows graphs showing the tissue weight of the mice in respective groups at the time of anatomy (Fig. 5A: liver weight, Fig. 5B: epididymis fat testis body fats (EPI WAT) weight, Fig. 5C: brown adipose tissue (BAT) weight).
Fig. 6 shows graphs showing the fasting blood glucose (FBC) (Fig. 6A), insulin concentration (Fig. 6B), and insulin resistance index (HOMA-R) (Fig. 6C), of the mice in respective groups at 12 weeks after the start of the food ingestion experiment.
Fig. 7 is a graph showing the blood triglyceride concentration of the mice in respective groups at 12 weeks after the start of the food ingestion experiment.
Fig. 8 shows graphs showing the blood leptin concentration (Fig. 8A), blood adiponectin concentration (Fig. 8B), blood TNF-α concentration (Fig. 8C), and blood IL-1β concentration (Fig. 8D), of the mice in respective groups at 12 weeks after the start of the food ingestion experiment.
Fig. 9 shows graphs showing the blood AST value (Fig. 9A), blood ALT value (Fig. 9B), and liver triglyceride concentration (Fig. 9C), of the mice in respective groups at 12 weeks after the start of the food ingestion experiment, and Fig. 9D shows photographs of the appearance of the liver of the mice in respective groups at 12 weeks after the start of the food ingestion experiment.
Fig. 10 shows graphs showing the liver AMPK phosphorylation (upper panel: Western blotting band) (Fig. 10A), and liver ACC phosphorylation (upper panel: Western blotting band) (Fig. 10B), of the mice in respective groups at 12 weeks after the start of the food ingestion experiment.
Fig. 11 shows graphs showing the comparison of the expression of liver fatty acid synthesis-related genes (Fig. 11A), and comparison of the expression of fatty acid oxidation-related genes (Fig. 11B), of the mice in respective groups at 12 weeks after the start of the food ingestion experiment.
Fig. 12 shows graphs showing the blood lipoperoxide concentration (Fig. 12A), liver lipoperoxide concentration (Fig. 12B), Mn-SOD gene expression (Fig. 12C), CuZn-SOD gene expression (Fig. 12D), Catalase gene expression (Fig. 12E), and GP-x (glutathione peroxidase) gene expression (Fig. 12F), of the mice in respective groups at 12 weeks after the start of the food ingestion experiment.
Fig. 13 shows graphs showing the F4/80 gene expression (Fig. 13A), TNF-α gene expression (Fig. 13B), and IL-1β gene expression (Fig. 13C) in the liver of the mice in respective groups at 12 weeks after the start of the food ingestion experiment.
Fig. 14 shows graphs showing the p38 MAPK phosphorylation (Fig. 14A), NF-κB phosphorylation (Fig. 14B), and JNK phosphorylation (Fig. 14C) in the liver of the mice in respective groups at 12 weeks after the start of the food ingestion experiment.
Fig. 15 shows graphs showing the gene expression of the liver fibrosis index group (αSMA gene expression (Fig. 15A), PAI1 gene expression (Fig. 15B), TGF-β1 gene expression (Fig. 15C), and Type1 collagen gene expression (Fig. 15D)), and the protein amount (F4/80 protein amount (Fig. 15E), αSMA protein amount (Fig. 15F), and TGF-β1 protein (Fig. 15G)) of the mice in respective groups at 12 weeks after the start of the food ingestion experiment.
Fig. 16 shows photographs of the liver tissue of mice in respective groups (A: normal diet only, B: normal diet+3% taxifolin, C: high-fat diet only, D: high-fat diet+3% taxifolin) stained with hematoxylin and eosin (HE) 12 weeks after the start of the food ingestion experiment (scale bar: 100 µm inside the white frame, 500 µm for others).
Fig. 17 shows photographs of the liver tissue of mice in respective groups (A: normal diet only, B: normal diet+3% taxifolin, C: high-fat diet only, D: high-fat diet+3% taxifolin) stained with azan (Azan) 12 weeks after the start of the food ingestion experiment (scale bar: 50 µm).
Fig. 18 shows photographs of the liver tissue of mice in respective groups (A: normal diet only, B: normal diet+3% taxifolin, C: high-fat diet only, D: high-fat diet+3% taxifolin) stained with sirius red (SiriusRed) 12 weeks after the start of the food ingestion experiment (scale bar: 100 µm).
Fig. 19 shows photographs of the liver tissue of mice in respective groups (A: normal diet only, B: normal diet+3% taxifolin, C: high-fat diet only, D: high-fat diet+3% taxifolin) immunohistochemically stained with anti-F4/80 antibody 12 weeks after the start of the food ingestion experiment (scale bar: 100 µm).

### [Description of Embodiments]

The terms used in the present specification are defined as follows.

In the present specification, "taxifolin" is a compound represented by the following formula (1):

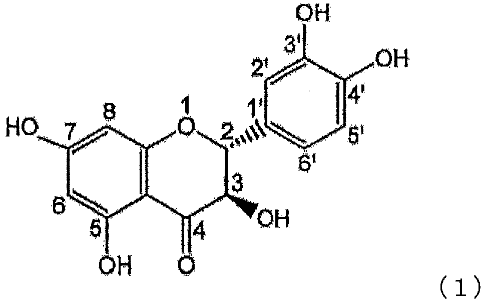

Taxifolin is one type of flavonoid that accounts for the most part (not less than 90%) of the active ingredient of diquvertin, which is a bioflavonoid complex isolated from the xylem of dahurian larch or Larix sibirica.

In the present specification, a salt of taxifolin is, for example, a salt that can be formed easily by reacting with a pharmaceutically acceptable base. Examples of such salt include alkali metal salts such as sodium salt, potassium salt and the like, and the like.

Taxifolin derived from Larix sibirica is an optically active form ((+)-taxifolin). Taxifolin in the present invention encompasses not only (+)-taxifolin but also its enantiomer (-)-taxifolin, a compound having low optical purity (both optically pure enantiomers are mixed in a suitable mixing ratio), and racemates. Taxifolin in the present invention also encompasses labeled forms, that is, compounds in which one or more atoms constituting taxifolin are labeled with isotope (e.g., ²H, ³H, ¹¹C, ¹³C, ¹⁴C, ¹⁵N, ¹⁸O, ¹⁸F, ³⁵S, etc.).

An optically active form of taxifolin or a salt thereof in the present invention can be produced by chemical synthesis according to a method known per se, or by extraction and/or purification from the xylem of dahurian larch or Larix sibirica. To be specific, an optically active form can be obtained by using an optically active synthesis intermediate, or optical resolution of a racemate of the final product according to a conventional method (e.g., J. Jacques et. al., "Enantiomers, Racemates and Resolution, John Wiley And Sons, Inc."). In addition, (+)-taxifolin is commercially available, and a commercially available product may also be used as it is.

Taxifolin or a salt thereof in the present invention may be a crystal, and a single crystal form and a mixture of crystal forms are encompassed in the taxifolin or a salt thereof. Crystals can be produced by crystallization by applying a crystallization method known per se.

Taxifolin or a salt thereof in the present invention may also encompass solvates thereof. The solvate thereof is a taxifolin or a salt thereof coordinated with a molecule of the solvent, and also encompasses hydrates. For example, hydrate, ethanol solvate, dimethyl sulfoxide solvate and the like of taxifolin or a salt thereof can be mentioned.

In the present specification, "nonalcoholic fatty liver disease (NAFLD)" is, according to the NAFLD/NASH guideline for the clinical practice of 2014 (edited by The Japanese Society of Gastroenterology, Nankodo Co., Ltd.; non-patent document 1), a pathological condition in which fatty liver is observed by tissue diagnosis or image diagnosis, and other hepatic diseases alcoholic hepatopathy and the like are excluded. NAFLD is classified into nonalcoholic fatty liver (hereinafter referred to as "NAFL") developed on the basis of histologically large droplet (such as a macrovesicular fat as the nucleus is unevenly distributed) liver fat degeneration and considered to show little progression, and nonalcoholic steatohepatitis (NASH). NAFL does not show a hepatocyte disorder (balloon-like degeneration) or fibrosis.

In the present specification, "nonalcoholic steatohepatitis (NASH)" is, according to the NAFLD/NASH guideline for the clinical practice of 2014 (edited by The Japanese Society of Gastroenterology, Nankodo Co., Ltd.; non-patent document 1), a pathological condition that is progressive and can be the origin of cirrhosis and hepatocellular cancer, in which balloon-like degeneration (hepatocyte disorder) and fibrosis of hepatocytes accompanied by inflammation are observed in addition to macrovesicular steatosis of hepatocytes (liver fat degeneration). It is classified into mild, moderate, and severe according to the degree of fattening, changes in hepatocytes such as balloon-like degeneration and the like, the degree of inflammation, and the degree of progression and aggravation of fibrosis. While there are cases of NASH in which fibrosis is not observed in the initial stage, caution is required because fat droplets decrease or disappear in many cases due to the progression of fibrosis. According to the diagnostic criteria of Younossi et al. (Hepatology, 2011; 53: 1874-1882), the degree of fibrosis determines the severity in the pathological diagnosis of NASH. The liver fibrosis inhibiting agent of the present invention is particularly effective in the prophylaxis and/or treatment of NASH accompanied by liver fibrosis (moderate or higher NASH).

In the present specification, "liver fibrosis" is a basic pathological condition of diffuse liver disease (acute hepatitis, chronic hepatitis, cirrhosis, etc.), and the degree thereof correlates with the progression of the pathological condition. Advanced liver fibrosis means transition to cirrhosis and suggests a high risk of hepatocellular cancer development. It has been reported that, in various hepatic diseases, the degree of liver fibrosis strongly correlates with prognosis including overall mortality (e.g., J. Gastroenterol., 2016; 51: 380-389 etc.). In addition, recent advances in viral hepatitis treatment have proved that liver fibrosis is a reversible pathological condition.

For the evaluation of liver fibrosis, it is also possible to measure the consistency of the liver non-invasively by using FibroScan, in addition to tissue diagnosis by liver biopsy. Recently, it has been reported that a characteristic tissue image called crown-like structure (CLS) is commonly formed in adipose tissue in obesity and NASH, and that tissue fibrosis progresses from here as the origin (e.g., Takayoshi Sugami, Research Report of the Uehara Memorial Foundation, 31 (2017), etc.).

In the present specification, the "liver fibrosis inhibiting agent" means a medicament that suppresses the progression of liver fibrosis when administered to a subject. The "liver fibrosis inhibiting agent" encompasses a medicament that improves hepatic fibrosis. Liver fibrosis to be the target of the "liver fibrosis inhibiting agent" of the present invention, is not particularly limited, but is preferably caused by hepatitis virus infection, NASH, or autoimmune hepatitis, more preferably NASH.

In the present specification, the "brown adipocyte activating agent" means a substance having the action of promoting induction of differentiation of brown adipocytes in the target white adipose tissue (that is, action of increasing the number of brown adipocytes), and/or the action of enhancing the expression of UCP1 by activating brown adipocytes, thereby promoting energy consumption.

The presence of brown adipocyte, changes in the number of brown adipocytes, and/or an increase in energy consumption in brown adipocytes can be evaluated by measuring the weight of brown adipose tissue and rectal temperature, measuring UCP1 mRNA expression and the amount of UCP1 protein, observing cells histologically, measuring accumulation of ¹⁸F-labeled glucose by PET, measuring cold-induced heat production, and the like.

In the present specification, "promote energy consumption" means enhancing energy consumption of brown adipocyte by heat production.

In the present specification, "promote the expression of UCP1" means at least one of the actions of promoting the expression level of the UCP1 gene in a subject, increasing the translation level of UCP1 protein, and activating UCP1. By promoting the expression level of UCP1, an antiobesity effect can be expected because it can promote lipolysis in white adipocytes and at the same time activate UCP1 to convert free fatty acids into heat and ultimately reduce body fat.

In the present specification, the "prophylaxis" includes prevention of the onset of diseases, delaying the onset of diseases, and prevention of the occurrence of pathological conditions. The "prophylactically effective amount" refers to a dose of the active ingredient which is sufficient to achieve the purpose of prophylaxis.

In the present specification, the "treatment" includes cure of a disease, improvement of the pathological condition of a disease (e.g., one or a plurality of symptoms), and suppression of the progression of a disease (severity). The "therapeutically effective amount" refers to a dose of the active ingredient which is sufficient to achieve the purpose of treatment. Therefore, "improvement" is a concept encompassed in the "treatment".

In the present specification, the "target" means a subject to which a drug or food containing an effective amount of an active ingredient necessary for the prophylaxis and/or treatment (or improvement) of a disease or the pathological condition of the disease is administered or fed. The "subject" includes, for example, human or non-human animal (particularly mammal (e.g., mouse, rat, guinea pig, hamster, rabbit, cat, dog, bovine, sheep, monkey, etc.)).

In the present specification, the "dietary therapy" is a therapy designed to lose weight by ingesting a low-calorie diet with a limited carbohydrate energy ratio of 50 - 60% and a limited fat energy ratio of 20 - 25%. Examples of the specific goal of body weight loss include a body weight loss of not less than 3% of the current body weight in the case of obesity with a high BMI (Body Mass Index) of 25 - 30, a body weight loss of 5 - 10% of the current body weight in the case of severe obesity with a BMI (Body Mass Index) of 35 or more (see obesity treatment guideline 2016), a body weight loss of not less than 7% in the case of NAFLD/NASH patients (see NAFLD/NASH guideline for the clinical practice of 2014), and the like.

In the present specification, the "exercise therapy" is, for example, a therapy to improve liver fattening by continuing aerobic exercise of about 50% of the maximum intensity (with light sweating) for 30 - 60 min at a time, 3 to 4 times a week for 4 - 12 weeks.

In the present specification, the "drug therapy" is a therapy designed to enhance the action and reduce side effects by using other drugs described below in combination.

In the present specification, the "iron removal therapy" is a therapy to reduce intake of excess iron that strains the liver. Specific examples of the iron removal therapy include therapeutic phlebotomy (therapy to draw 200 - 400 mL of blood at a time every 2 weeks), and iron-restricted diet (reduce iron in daily diet to not more than 6 - 7 mg).

### The pharmaceutical composition of the present invention (prophylactic and/or therapeutic agent of the present invention)

As shown in the below-mentioned Experimental Examples, since taxifolin has a superior liver fibrosis suppressive action (activation of liver AMP activated protein kinase (AMPK), suppression of expression of inflammatory cytokine in liver tissue, and reduction of fibrosis of liver tissue), and a brown adipocyte activation action (raising rectal temperature, and increased expression of UCP1 gene), taxifolin or a salt thereof, and a composition containing same as an active ingredient can be preferably used as a liver fibrosis inhibiting agent (hereinafter to be referred to as "the liver fibrosis inhibiting agent of the present invention") and/or a brown adipocyte activating agent (hereinafter to be referred to as "brown adipocyte activating agent of the present invention"). In addition, an anti-fibrosis effect on NASH, an antiobesity effect, glucose tolerance improvement, and insulin resistance improvement are observed in relation to the liver fibrosis suppressive action and the brown adipocyte activation action. Therefore, the liver fibrosis inhibiting agent and/or the brown adipocyte activating agent of the present invention are/is useful not only as a prophylactic and/or a therapeutic agent for various metabolic syndromes, but also a prophylactic and/or a therapeutic agent for NASH accompanied by fibrosis. Since little is known to date about a drug that significantly suppresses fibrosis of liver tissue, the medicament of the present invention can be an effective prophylactic and/or therapeutic agent for NASH with high severity.

In the following, a pharmaceutical composition containing taxifolin or a salt thereof as an active ingredient in the present specification is sometimes to be referred to as "the medicament of the present invention" or "the prophylactic and/or therapeutic agent of the present invention".

The medicament of the present invention may be either a medicament consisting of taxifolin or a salt thereof alone, or a medicament containing taxifolin or a salt thereof and a pharmaceutically acceptable carrier or the like. A prophylactically effective amount or therapeutically effective amount of the medicament of the present invention can be orally administered to a subject.

Examples of the pharmaceutically acceptable carrier include excipient (e.g., starch, lactose, sugar, calcium carbonate, calcium phosphate, etc.), binder (e.g., starch, gum arabic, carboxymethylcellulose, hydroxypropylcellulose, crystalline cellulose, etc.), lubricant (e.g., magnesium stearate, talc, etc.), disintegrant (e.g., carboxymethylcellulose, talc, etc.), solvent (e.g., water for injection, physiological brine, Ringer's solution, alcohol, propylene glycol, polyethylene glycol, sesame oil, corn oil, olive oil, cottonseed oil, etc.), solubilizing agent (e.g., polyethylene glycol, propylene glycol, D-mannitol, trehalose, benzoic acid benzyl, ethanol, tris aminomethane, cholesterol, triethanolamine, sodium carbonate, sodium citrate, salicylic acid sodium, sodium acetate, etc.), suspending agent (e.g., stearyltriethanolamine, sodium lauryl sulfate, lauryl aminopropionic acid, lecithin, benzalkonium chloride, benzethonium chloride, mono stearic acid glycerol and the like surfactant; polyvinyl alcohol, polyvinylpyrrolidone, carboxymethylcellulose sodium, methylcellulose, hydroxymethylcellulose, hydroxyethylcellulose, hydroxypropylcellulose and the like coating base material; polysorbates, polyoxyethylene hydrogenated castor oil, etc.), isotonicity agent (e.g., sodium chloride, glycerol, D-mannitol, D-sorbitol, glucose, etc.), buffering agent (e.g., phosphate, acetate, carbonate, citrate and the like buffer, etc.), thickener (e.g., sodium alginate, xanthan gum, chondroitin sulfuric acid sodium, polyvinyl alcohol, povidone, etc.), antiseptic (e.g., p-hydroxybenzoic acid esters, chlorobutanol, benzyl alcohol, phenethyl alcohol, dehydroacetic acid, sorbic acid, etc.), antioxidant (e.g., sulfite, ascorbate, etc.), colorant (e.g., water-soluble food tar color (e.g., food colors such as Food Color Red Nos. 2 and 3, Food Color yellow Nos. 4 and 5, Food Color Blue Nos. 1 and 2, and the like), water-insoluble lake pigment (e.g., aluminum salts of the aforementioned water-soluble food tar colors), natural dye (e.g., β-carotene, chlorophyll, red iron oxide), etc.), sweetening agent (e.g., saccharin sodium, dipotassium glycyrrhizinate, aspartame, stevia, etc.) and the like.

The medicament of the present invention (that is, the prophylactic and/or therapeutic agent of the present invention) is mixed with the above-mentioned various components, and then the mixture can be formulated as, for example, a preparation for oral administration such as tablet, pill, capsule (including hard capsule, soft capsule, microcapsule), powder, granule, fine granules, troche, or liquid (including syrup, emulsion, suspension) or the like by means known per se.

Food compositions in the form of tablet, granule, and fine granule may be coated with the aforementioned coating base material by a method known per se, for the purpose of masking the taste, improving the light stability, improving the appearance, enteric property, and the like.

While the content of taxifolin or a salt thereof in the medicament of the present invention varies depending on the form of the preparation, it is generally about 10 - 100 wt%, preferably about 30 - 100 wt%, more preferably about 50 - 100 wt%, with respect to the whole preparation.

The dose (that is, prophylactically effective amount or therapeutically effective amount) of taxifolin or a salt thereof varies depending on the subject of administration, disease, symptoms, dosage form, administration route and the like. For example, in the case of human, when orally administered to an adult patient (body weight about 60 kg), the amount of taxifolin, which is the active ingredient, is generally 10 - 10000 mg, preferably 30 - 1000 mg, more preferably 50 - 500 mg, which amount can be administered once or in several divided doses per day, regardless of preprandial, postprandial, or inter cibos. The dosing period is not particularly limited.

Taxifolin or a salt thereof can be used in combination with other therapy (that is, the aforementioned dietary therapy, exercise therapy, drug therapy, iron removal therapy, surgical treatment). As a result, the prophylactic and/or treatment effect of taxifolin or a salt thereof can be potentiated. Among these, combination with dietary therapy and exercise therapy, or combination with drug therapy is particularly preferred.

Specific embodiment of the combination with a drug therapy is shown in the following.

Taxifolin or a salt thereof can be used in combination with other medicament (concomitant drug) as long as the efficacy thereof is not impaired. At this time, the administration period is not limited, and these may be administered to the subject at the same time or at different times. They can also be administered as a single preparation containing taxifolin or a salt thereof in combination with a concomitant drug.

The dose of the concomitant drug can be appropriately selected based on the dose used clinically. In addition, the mixing ratio of taxifolin or a salt thereof and the concomitant drug can be appropriately selected according to the subject of administration, administration route, target disease, symptom, kind of concomitant drug, and the like.

Examples of the concomitant drug when taxifolin or a salt thereof (or the brown adipocyte activating agent of the present invention) is used for the prophylaxis and/or treatment of NASH include insulin sensitizer, HMG-CoA reductase inhibiting agent, angiotensin II receptor antagonist, therapeutic drug for non-statin hypercholesterolemia, anti-oxidation drug, and the like.

As the "insulin sensitizer", for example, a thiazolidine derivative (e.g., pioglitazone hydrochloride, troglitazone, rosiglitazone or maleate thereof, GI-262570, JTT-501, MCC-555, YM-440, KRP-297, CS-011, FK-614, NN-622, AZ-242, BMS-298585, ONO-5816, LM-4156, BM-13-1258, MBX-102, GW-1536, etc.), biguanide (e.g., phenformin, metformin, buformin, etc.), and the like are used.

As the "HMG-CoA reductase inhibiting agent", for example, pravastatin, simvastatin, lovastatin, atorvastatin, fluvastatin, lipantil, cerivastatin, itavastatin, rosuvastatin, or a salt thereof (e.g., sodium salt, etc.), and the like are used.

As the "angiotensin II receptor antagonist", for example, candesartan cilexetil, losartan, eprosartan, valsartan, telmisartan, irbesartan, tasosartan, and the like are used.

As the "therapeutic drug for non-statin hypercholesterolemia", for example, ezetimibe and the like are used.

As the "anti-oxidation drug", for example, vitamin E, betaine, pentoxifylline, N-acetyl-L-cysteine, and the like are used.

### The food composition of the present invention

In the following, a food composition containing taxifolin or a salt thereof as an active ingredient in the present specification is sometimes to be referred to as "the food composition of the present invention".

The food composition of the present invention may be either a composition consisting of taxifolin or a salt thereof alone, or a composition containing taxifolin or a salt thereof and a food additive and the like. The food composition of the present invention may be any as long as it contains taxifolin or a salt thereof and the subject can orally ingest same. The kind, form and the like of the food composition are not particularly limited. In addition, as shown in the below-mentioned Experimental Examples, since taxifolin has a superior liver fibrosis suppressive action and a brown adipocyte activation action, the food composition of the present invention can be used for use for suppressing liver fibrosis, use for activating brown adipocyte, use for prophylaxis and/or improvement of NASH, and the like.

Examples of the food composition of the present invention include confectioneries such as drop, candy, ramune, gummy, chewing gum and the like; Western confectioneries such as cookie, cracker, biscuit, potato chips, bread, cake, chocolate, donut, pudding, jelly and the like; Japanese confectioneries such as rice cracker, yokan, daifuku, ohagi, manju̅, castella and the like; frozen dessert such as ice cream, ice candy, sherbet, gelato and the like; breads such as white bread, baguette, croissant and the like; noodles such as Japanese wheat noodles, Japanese buckwheat noodles, Chinese noodles, kishimen and the like; fish paste products such as boiled fish paste, chikuwa, fish sausage and the like; meat products such as ham, sausage, hamburger patty, corned beef and the like; seasonings such as salt, pepper, miso, soy sauce, sauce, dressing, mayonnaise, ketchup, sweetener (e.g., sugar, honey, powdered corn syrup, starch syrup, jam, marmalade, etc.), spices (e.g., mustard, pepper, etc.); teppanyaki foods such as Akashiyaki, Takoyaki, Monjayaki, Okonomiyaki, Yakisoba, and Yakiudon; dairy products such as cheese, butter, margarine, and yogurt; various side dishes such as natto, deep-fried tofu, tofu, konnyaku, dumplings, pickles, tsukudani, gyoza, shumai, croquettes, sandwiches, pizza, hamburger, and salads; livestock products such as beef, pork, and chicken; marine products such as shrimp, scallops, clams, and kelp; various powders of vegetables, fruits, plants, yeast, algae, etc.; powdered solidified oils and flavors (vanilla, citrus, bonito, etc.); beverages, and the like.

Examples of the beverages include food and drink such as soup, miso soup and the like; powder food and drink such as instant coffee, instant tea, instant milk, instant soup, instant miso soup and the like; whiskey, bourbon, spirits, liqueurs, wine, fruit wine, Japanese sake, Chinese sake, shochu, beer, non-alcoholic beer with alcohol content of 1% or less, alcoholic drinks such as low-malt beer, shochu highball and the like; beverages with fruit juice (e.g., apple, tangerine, grape, banana, pear, plum juice, etc.), beverages with vegetable juice (e.g., tomato, carrot, celery, cucumber, watermelon vegetable juice, etc.), beverages with fruit and vegetable juice, soft drinks, milk, soy milk, milk beverages, drink-type yogurt, coffee, cocoa, tea beverages (black tea, green tea, barley tea, brown rice tea, sencha, gyokuro tea, hojicha, oolong tea, turmeric tea, pu'erh tea, rooibos tea, rose tea, kiku tea, herbal tea (e.g., mint tea, jasmine tea), etc.), nutritional drinks, sports drinks, mineral water, and other non-alcoholic beverages and the like.

Preferable examples of such food composition include jelly, tea drinks, alcoholic drinks, drop, candy, ramune, cookie, cracker, biscuit, chocolate, cheese, butter, margarine, chewing gum and the like.

The food composition of the present invention may be prepared as functional food, health food, food for specified health uses, food for special dietary uses (e.g., food for sick people), supplement or the like, and preferably prepared as food for specified health uses, food for special dietary uses, or supplement.

Examples of the form of the food composition of the present invention include tablet, pill, capsule (including hard capsule, soft capsule, microcapsule), powder, granule, fine granule, troche, or liquid (including syrup, emulsion, suspension) and the like, and tablet or capsule is preferred.

The food composition of the present invention particularly preferably has the form of tablet or capsule of food for specified health uses, food for special dietary uses, or supplement.

In the present specification, supplement means not only nutritional supplements, nutrient function foods, etc., to supplement nutrients, etc., but also health supplements, health function foods, etc., that have functions useful for maintaining, restoring, and promoting health (especially functions to prevent and/or improve NASH) and the like.

The food composition of the present invention can be produced, for example, by adding taxifolin or a salt thereof to a food by a known method. Specifically, for example, a tablet food composition can be produced, for example, by adding and mixing taxifolin or a salt thereof, and materials such as excipient (e.g., lactose, sucrose, mannitol, etc.), sweetening agent, flavoring agent and the like, and molding them into the form of a tablet by applying pressure with a tableting machine press, or the like. Where necessary, other materials (e.g., vitamins such as vitamin C and the like, minerals such as iron, other additives such as dietary fiber and the like) may also be added. A capsule food composition can be produced, for example, by filling a liquid, suspension, paste, powdery, or granular food composition containing taxifolin or a salt thereof in a capsule, or by packaging same with a capsule base.

The food composition of the present invention can contain a physiologically acceptable carrier and the like as long as they do not inhibit the effects of the present invention, in addition to food material, food additive, various nutrients, vitamins, flavoring substance (e.g., cheese, chocolate, etc.) and the like used generally. As the physiologically acceptable carrier and the like, conventionally-used various organic or inorganic carrier substances are used, and excipient, binder, disintegrant, lubricant, colorant, sweetening agent, antiseptic, antioxidant, thickener, emulsifier and the like can be mentioned. As the food additive, colorant, sweetening agent, antiseptic, antioxidant, flavoring agent and the like can be mentioned. As other materials, for example, minerals such as iron and the like, dietary fibers such as pectin, carrageenan, mannan and the like, and the like may be contained.

Examples of the excipient, binder, disintegrant, lubricant, solvent, solubilizing agents, suspending agent, buffering agent, thickener, colorant, sweetening agent, antiseptic, and antioxidant include those similar to the ones used for the aforementioned medicament of the present invention.

Vitamins may be water-soluble or liposoluble and, for example, palmitic acid retinol, tocopherol, bisbentiamine, riboflavin, pyridoxine hydrochloride, cyanocobalamin, sodium ascorbate, cholecalciferol, nicotinamide, calcium pantothenate, folic acid, biotin, choline bitartrate and the like can be mentioned.

Food compositions in the form of tablet, granule and fine granule may be coated with a coating base material by a method known per se for the purposes of masking of taste, improvement of light stability, improvement of appearance, enteric property, and the like. Examples of the coating base material include those similar to the ones used for the aforementioned medicament of the present invention, and the coating can be performed in the same manner.

The content of taxifolin or a salt thereof in the food composition of the present invention is about 0.1 - 50 wt%, preferably about 0.5 - 30 wt%, more preferably about 1 - 20 wt%, with respect to the whole food composition.

The thus-obtained food composition is safe, and thus can be continuously given to the subject, particularly preferably human.

The ingestion amount of the food composition of the present invention may be within the range of an effective amount of taxifolin or a salt thereof that activates brown adipocytes, or an effective amount that prevents and/or improves NASH. For example, when the food composition of the present invention is ingested by an adult for the purpose of preventing and/or improving NASH, the ingestion amount of taxifolin or a salt thereof varies depending on the subject who ingests the composition, the manner of ingestion, the amount of food ingestion, and the like. The ingestion amount of taxifolin, which is the active ingredient, is generally 10 - 10000 mg per day, preferably 30 - 1000 mg per day, more preferably 50 - 500 mg per day, and the amount can be ingested once or in several divided doses per day. In addition, the above-mentioned ingestion amount is preferable from the aspect that the effect is expressed without affecting the preference or the amount of food ingested. Similar amounts can be ingested even when the subject is other animal.

Furthermore, the food composition of the present invention may be used alone, or may be used in combination with other therapy (that is, the aforementioned dietary therapy, exercise therapy, drug therapy, iron removal therapy, surgical treatment). Specifically, for example, it can be used in combination with other pharmaceutical compositions, food compositions, or feeds having a liver function improving effect. By combining with other therapy, liver fibrosis suppressive effect, brown adipocyte activation effect (specifically, for example, energy consumption promoting effect), prophylactic and/or improving effect on NASH (particularly, NASH accompanied by liver fibrosis), and the like can be further enhanced.

In addition, the food composition of the present invention encompasses those under classifications of health food, functional food, food for specified health uses, health-promoting food, food with a disease risk reduction label, or food for special dietary uses (e.g., food for sick people). Examples of the disease risk reduction label include labeling to the effect that the product is for reducing the risk of liver fibrosis, activating brown fat cells, promoting energy metabolism, or the like. Therefore, the food composition of the present invention is, for example, a food or drink with an indication that it contains taxifolin or its salt and is for reducing the risk of liver fibrosis, or with an indication that it contains taxifolin or its salt and activates brown fat cells and promotes energy metabolism.

The functional labeling on these food compositions can be on the body of the product, the container, the packaging, the instructions, the package insert, or any promotional material.

### [Example]

The present invention is explained in more detail in the following by referring to Experimental Examples and Formulation Examples, which do not limit the present invention. The present invention may be modified without departing from the scope of the invention. The reagents, apparatuses, materials and the like used in the present invention are commercially available unless particularly indicated.

The taxifolin used in the following Experimental Examples was purchased from Ametis JSC (Russia) and used as was.

### Experimental Example 1: Effect of taxifolin intake on NASH in a mouse model of high-fat diet-induced obesity

### (1) method

### (1-1) food ingestion experiment

C57BL/6J mice were used as experimental animals. They were purchased at the age of 7 weeks from CLEA Japan, Inc. and randomly divided into four groups at the age of 8 weeks after 1 week of acclimation as shown in Table 1 below.

**[Table 1]**

| | normal diet (body weight: g) | high-fat diet (body weight: g) |
|---|---|---|
| without taxifolin | 5 mice (22.2±0.4) | 5 mice (22.8±0.2) |
| with taxifolin (3%) | 5 mice (22.8±0.2) | 5 mice (22.6±0.3) |

| | | |
|---|---|---|
| mean±standard error | | |

They were allowed to eat and drink freely, and their food intake and body weight were measured once a week. Fasting blood glucose was measured on the first day of the feeding experiment and once every 4 weeks thereafter (at 4, 8, and 12 weeks after the start of the feeding). At the end of 12 weeks, an intraperitoneal glucose tolerance test (IPGTT) was performed to analyze glucose tolerance. Rectal temperature was also measured at 8 and 12 weeks follow-up (Table 2).

| [Table 2] | mouse age | | | | | | | | | | | | | | |
|---|---|---|---|---|---|---|---|---|---|---|---|---|---|---|---|
| experimental group | n | 7 week-old | 8 week-old | 9 week-old | 10 week-old | 11 week-old | 12 week-old | 13 week-old | 14 week-old | 15 week-old | 16 week-old | 17 week-old | 18 week-old | 19 week-old | 20 week-old |
| | | | experiment start | → → → → → → → → → → → → → → → → → → | | | | | | | | | | | autopsy |
| taxifolin-unmixed normal diet intake group | 5 | purchase ↓ acclimation | taxifolin-unmixed normal diet, free to eat | | | | | | | | | | | | |
| 3% taxifolin-mixed normal diet intake group | 5 | | 3% taxifolin-mixed normal diet, free to eat | | | | | | | | | | | | |
| taxifolin-unmixed high-fat diet intake group | 5 | | taxifolin-unmixed high-fat diet, free to eat | | | | | | | | | | | | |
| 3% taxifolin-mixed high-fat diet intake group | 5 | | 3% taxifolin-mixed high-fat diet, free to eat | | | | | | | | | | | | |
| body weight measurement | | | O | O | ○ | ○ | ○ | O | O | ○ | O | O | O | O | O |
| intake amount measurement | | | ○ | O | ○ | ○ | O | O | O | O | O | O | ○ | ○ | O |
| fasting blood glucose measurement | | | ○ | | | | ○ | | | | ○ | | | | ○ |
| rectal temperature measurement | | | | | | | | | | | O | | | | O |
| intraperitoneal glucose tolerance test | | | | | | | | | | | | | | | O |

| | | | | | | | | | | | | | | | |
|---|---|---|---|---|---|---|---|---|---|---|---|---|---|---|---|
| * high-fat diet: 60% calorie ratio high-fat diet sample | | | | | | | | | | | | | | | |

The last fasting blood glucose was measured 5 days after the day of IPGTT, and all blood samples were collected under ether anesthesia, then the mice were euthanized by cervical dislocation, and samples were collected and partially weighed as shown in Table 3 below.

**[Table 3]**

| blood | serum collection (3000 rpm, 10 min), blood cells after serum collection are also stored (-80°C) | | | |
|---|---|---|---|---|
| tissue sample | wet weight measurement | after liquid nitrogen, stored at - 80°C (for DNA, RNA, protein analysis) | 4% paraformaldehyde fixation | |
| | | | frozen embedding | paraffin embedding |
| tibialis anterior muscle (white muscle fast muscle) | O (one side) | O | | |
| extensor digitorum longus (white muscle · fast muscle) | O (one side) | ○ | | |
| soleus muscle (red muscle · slow muscle) | O (one side) | O | | |
| gastrocnemius (red-white mixed muscle) | O (one side) | O | | |
| brain | O | O | | |
| subcutaneous fat | - | O | O | |
| epididymis fat | ○ (one side) | O | O | |
| liver | O | O | | O |
| kidney | O (one side) | O | | O |
| pancreas | - | - | | O |
| mesenteric fat | - | ○ | O | |
| brown adipose tissue | O | O | O | |
| feces | | O | | |
| small intestine, cecum, colon | O | O | | |

### (1-2) Sample preparation (liver only) and storage

Liver samples were partially trimmed for tissue observation, washed, immersed in 4% paraformaldehyde, and fixed at 4°C overnight. The fixed samples were washed, dehydrated in an ascending ethanol series, and paraffin-embedded.

The remaining liver samples were flash frozen in liquid nitrogen and stored at -80°C until ready for each experiment.

### (1-3) Analysis

(i) Blood concentration and biochemical test
   - insulin concentration: Ultra-sensitive mouse insulin ELISA kit (Morinaga Institute of Biological Science, Inc.)
   - leptin concentration: mouse/rat leptin ELISA kit (Morinaga Institute of Biological Science, Inc.)
   - adiponectin concentration: mouse/rat adiponectin ELISA kit (Otsuka Pharmaceutical Co., Ltd.)
   - Triglyceride: Lab Assay Kit (Wako)
   - TNF-α, IL-1β concentration: ELISA kit (Proteintech)
   - ALT, AST levels: Assay Kit (Funakoshi Co., Ltd.)
   - glucose tolerance: The area under the blood glucose curve (AUC) was calculated from the blood glucose fluctuation curve from fasting blood glucose level to 120 min after ingestion using the trapezoidal formula.
(ii) Oxidation stress
   - Blood and liver tissue lipid peroxidation: Lipid Peroxidation (MDA) Colorimetric Assay Kit (BioVision, Inc.)
   - Anti-oxidation enzyme (Mn-SOD, CuZn-SOD, Catalase, GPx) gene expression: Real-time Quantitative RT PCR
(iii) Liver fatty acid synthesis/fatty acid oxidation
   - Srebp1c, Lxrα, FAS, Scd1, PPARα, Cpt1a gene expression: Real-time Quantitative RT PCR
(iv) AMPK/ACC activity
   - AMPK phosphorylation, ACC phosphorylation: Western blotting
(v) Liver tissue inflammation index/inflammation induction signal pathway activity
   - F4/80, TNF-α, IL-1β gene expression: Real-time Quantitative RT PCR
   - P38 MAPK phosphorylation, NF-κB p65 phosphorylation, JNK phosphorylation: Western blotting
(vi) Liver fibrosis index
   - αSMA, PAIl, TGF-β, 1-type Collagen gene expression: Real-time Quantitative RT PCR
   - F4/80, αSMA, TGF-β1 protein amount: Western blotting
(vii) Histological analysis of the liver
   Observation of morphology and fat accumulation: Hematoxylin Eosin (HE) staining
   Observation of fibrosis: Azan staining, SiriusRed staining, immunohistochemistry (anti-F4/80 antibody)
(viii) Analysis of brown adipocytes and UCP1
   Target tissue: Brown adipose tissue
   Target: UCP1 gene
   Means of analysis: real-time RT PCR analysis

### (2) Results

### (i) Weight change (1/week measurement)

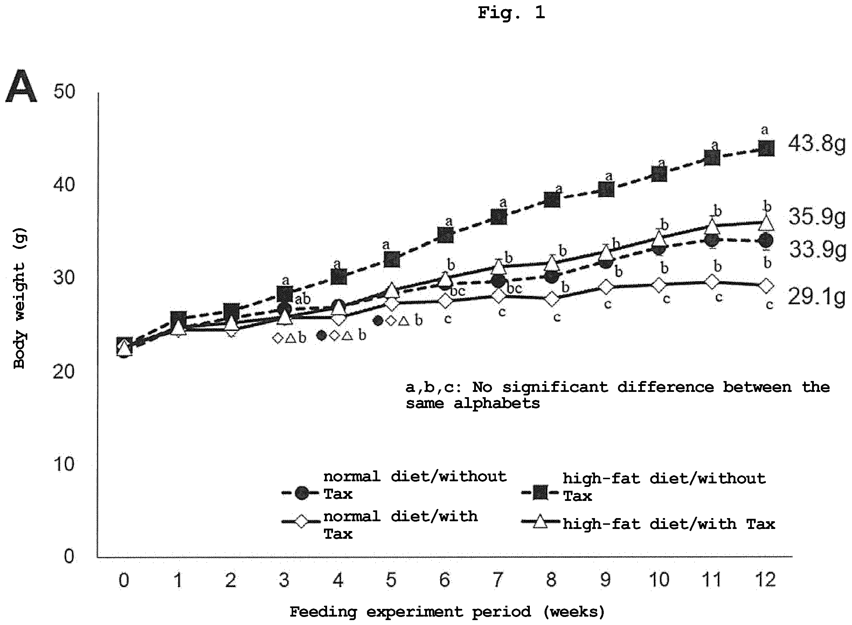

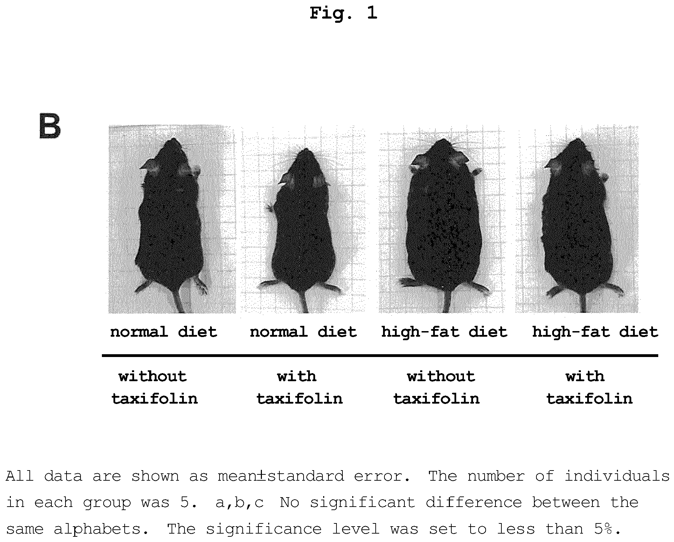

From the results shown in Fig. 1, a significant difference (increase) in body weight was observed in the high-fat diet only group (high-fat diet/without Tax) at 3 weeks after the start of the feeding experiment, and a significant difference was observed between the high-fat diet only group and the other groups at 4 weeks. In addition, a significant difference in weight loss was confirmed in the normal diet + taxifolin group (normal diet/with Tax) at 6 weeks after the start of the feeding experiment, and a significant difference in weight loss was confirmed in the normal diet + taxifolin group and all other groups at 8 weeks. At 12 weeks after the start of the feeding experiment, significant weight gain was observed in the high-fat diet-only group. In contrast, the weight of the high-fat diet plus taxifolin group (high-fat diet with Tax) was similar to that of the normal diet only group (normal diet without Tax), and no weight gain was observed in the normal diet plus taxifolin group compared to the other groups.

### (ii) Food intake (measured per week)

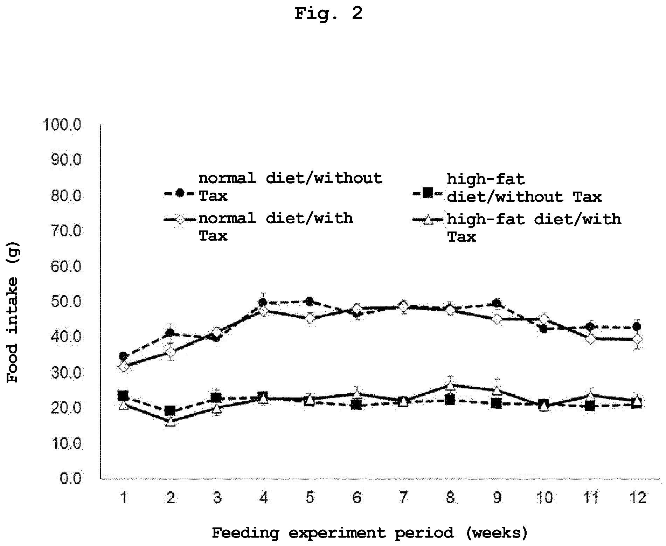

According to the results shown in Fig. 2, there was no difference in food intake between the normal diet only group (normal diet/without Tax) and the normal diet + taxifolin group (normal diet/with Tax) during the feeding period, and there was no difference in food intake between the high-fat diet only group (high-fat diet/without Tax) and the high-fat diet + taxifolin group (high-fat diet/with Tax). The daily intake of taxifolin was as follows at 12 weeks after the start of the feeding experiment.

High-fat diet + taxifolin intake group (high-fat diet with Tax): about 100 mg/about 36 g BW/animal/day

### (iii) Fasting blood glucose (measured at 1/4 week) and glucose tolerance

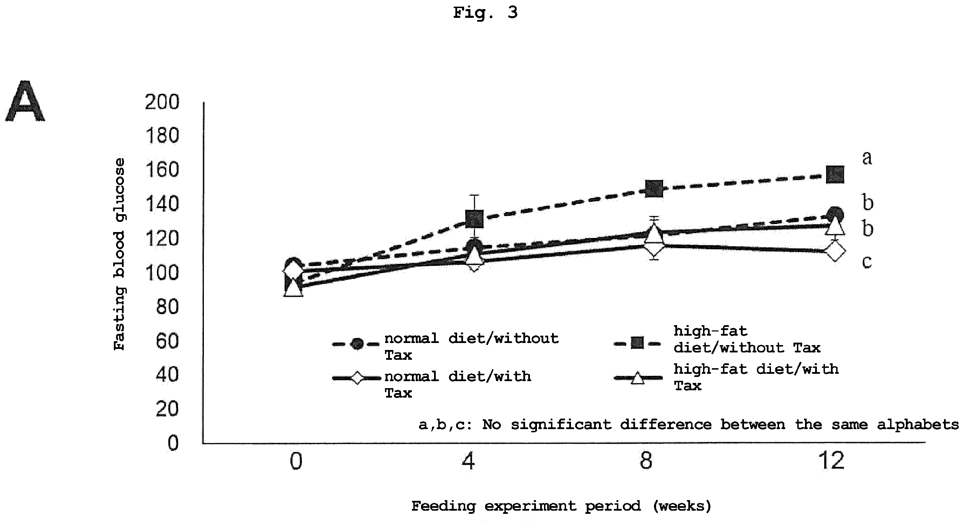

According to Fig. 3A, fasting blood glucose at 12 weeks after the start of the experiment was significantly higher in the high-fat diet only group (high-fat diet/without Tax) and significantly lower in the normal diet + Taxifolin group (normal diet/with Tax).

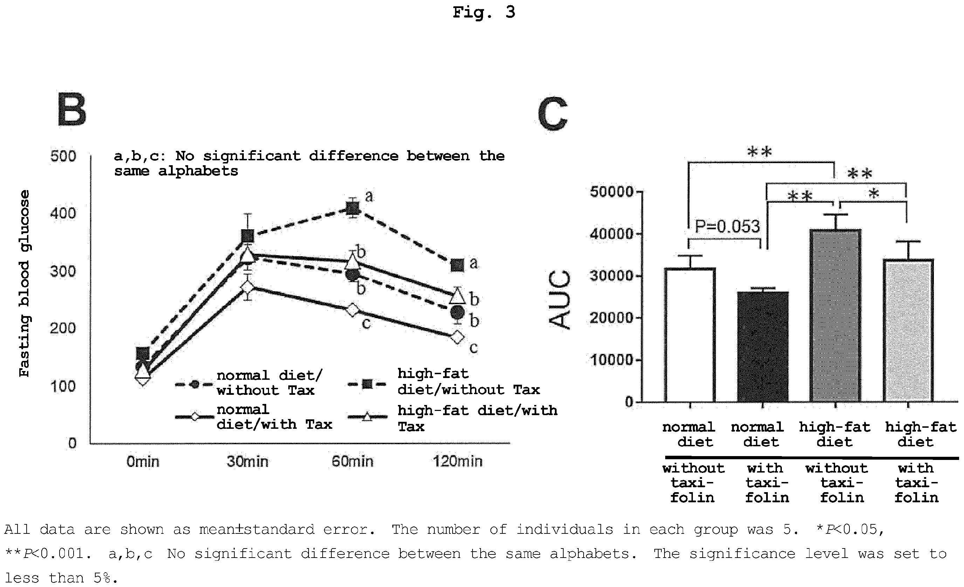

According to Fig. 3B, the blood glucose level at 60 min after the start of intraperitoneal glucose tolerance test was significantly higher in the high-fat diet only group (high-fat diet/without Tax) and significantly lower in the normal diet + taxifolin group (normal diet/with Tax).

According to Fig. 3C, AUC was also significantly decreased in the high-fat diet + taxifolin group (with normal diet/Tax) compared to the high-fat diet only group (without high-fat diet/Tax).

### (iv) Rectal temperature (at 8 and 12 weeks after the start of the feeding experiment)

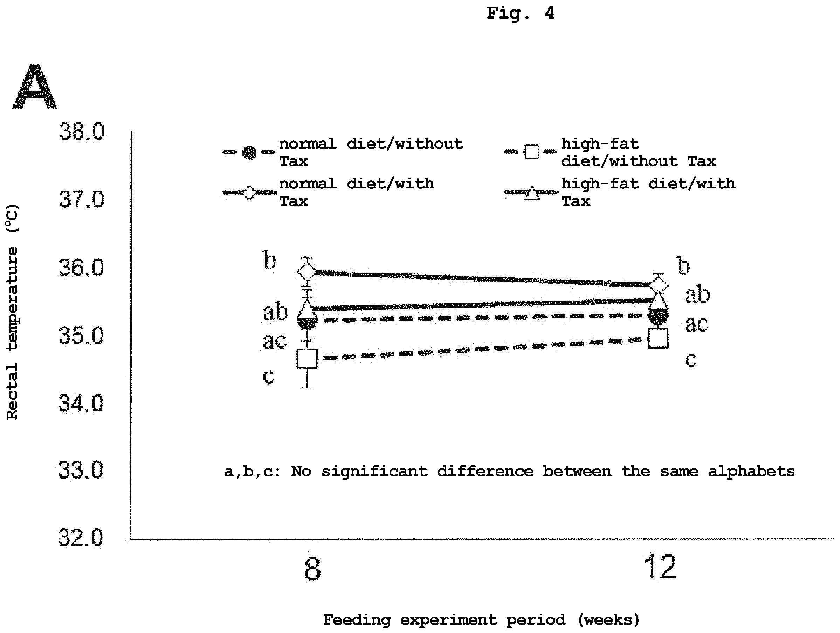

According to Fig. 4A, rectal temperature at 8 weeks after the start of the experiment was significantly lower in the high-fat diet only group (high-fat diet/without Tax) and significantly higher in the normal diet + Taxifolin group (normal diet/with Tax). This trend was also observed for rectal temperature at 12 weeks. This suggests that taxifolin activates brown adipocytes, which in turn enhances energy consumption, resulting in an increase in rectal temperature.

### (v) UCP1 gene expression

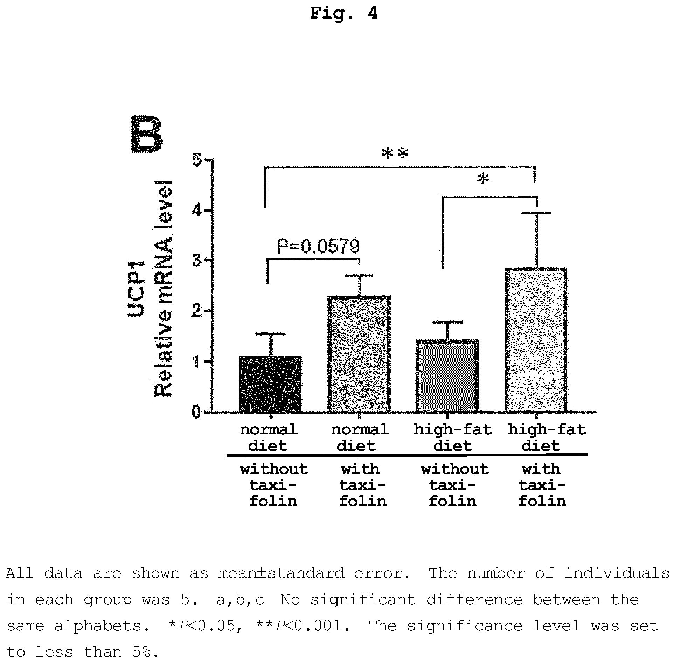

According to Fig. 4B, UCP1 gene expression was significantly increased in the high-fat diet plus taxifolin group (with taxifolin) compared to the high-fat diet only group (without taxifolin). This suggests that the expression of the UCP1 gene, which is involved in heat production, was enhanced by the feeding of taxifolin.

### (vi) Organ weight

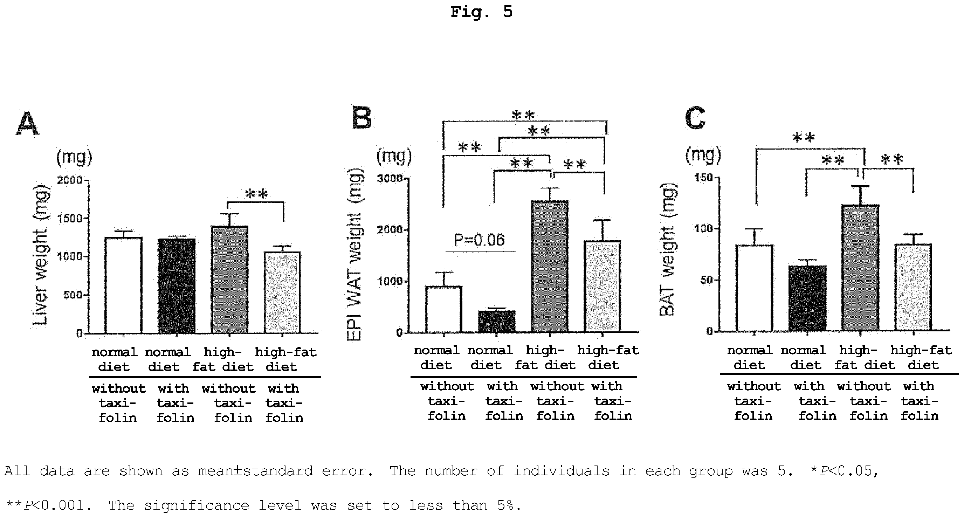

According to Fig. 5A, liver weight was confirmed to be significantly decreased in the high-fat diet + taxifolin group (with taxifolin) compared to the high-fat diet only group (without taxifolin).

According to Fig. 5B, epididymal fat weight (EPI WAT weight) was confirmed to be significantly decreased in the high-fat diet + taxifolin group (with taxifolin) compared to the high-fat diet only group (without taxifolin).

In addition, according to Fig. 5C, brown adipose tissue weight (BAT weight) was confirmed to be significantly decreased in the high-fat diet plus taxifolin group (with taxifolin) compared to the high-fat diet only group (without taxifolin).

### (vii) Blood insulin concentration/insulin resistance index (HOMA-R)

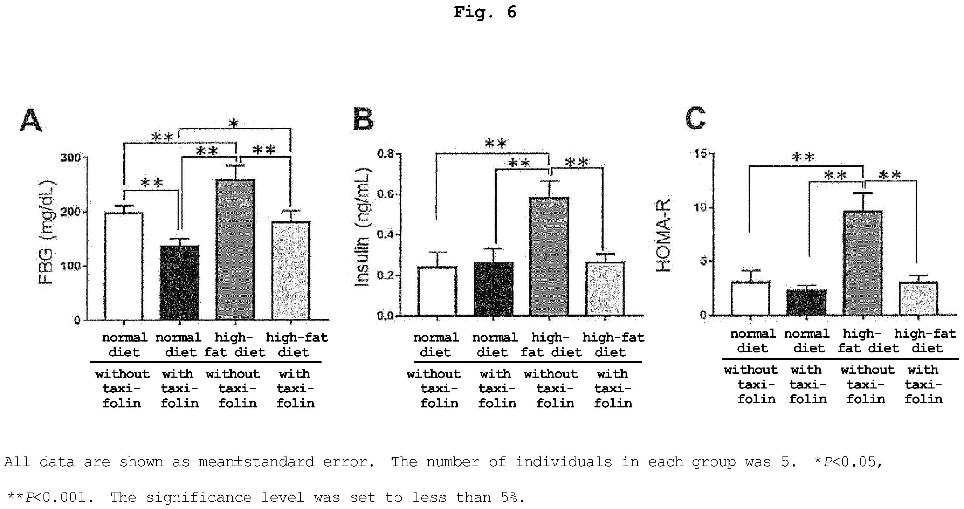

According to Fig. 6A, it was confirmed that fasting blood glucose after 12 weeks of the feeding experiment significantly decreased in the high-fat diet + taxifolin group (with taxifolin) compared to the high-fat diet only group (without taxifolin), and also significantly decreased in the normal diet + taxifolin group (with taxifolin) compared to the normal diet only group (without taxifolin).

According to Fig. 6B, it was confirmed that blood insulin concentration was significantly decreased in the high-fat diet + taxifolin group (with taxifolin) compared to the high-fat diet only group (without taxifolin).

According to Fig. 6C, insulin resistance index (HOMA-R) was also confirmed to be significantly decreased in the high-fat diet + taxifolin group (with taxifolin) compared to the high-fat diet only group (without taxifolin).

### (viii) Blood biochemistry

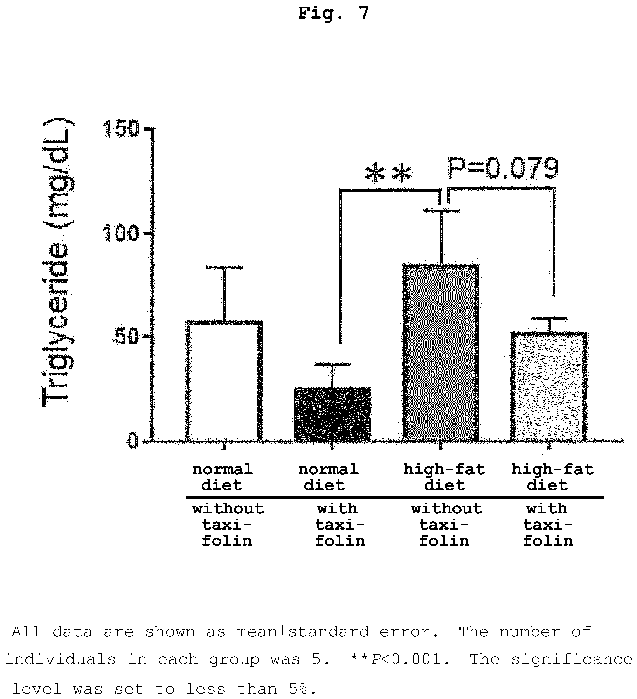

According to Fig. 7, blood triglyceride concentration showed a decreasing trend (P=0.079) in the high-fat diet + taxifolin group (with taxifolin) compared to the high-fat diet only group (without taxifolin).

### (ix) Blood adipocytokine concentration

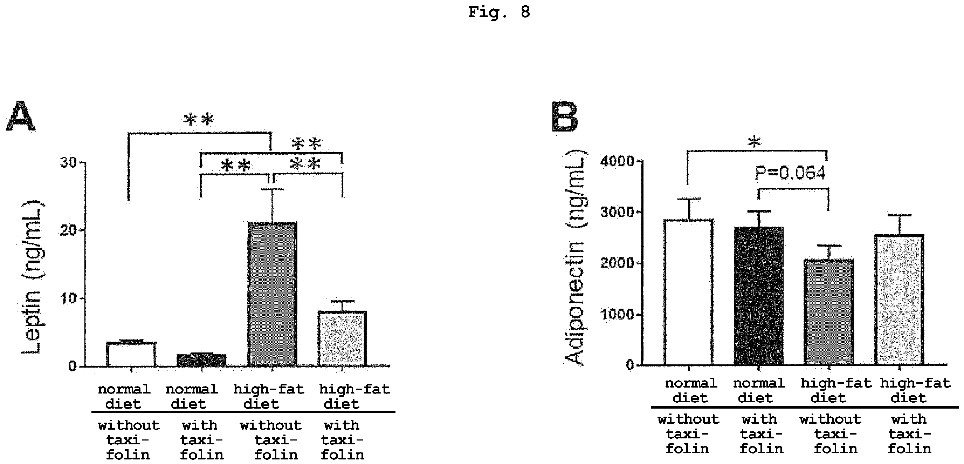

According to Fig. 8A, blood leptin concentration was confirmed to be significantly decreased in the high-fat diet + taxifolin group (with taxifolin) compared to the high-fat diet only group (without taxifolin).

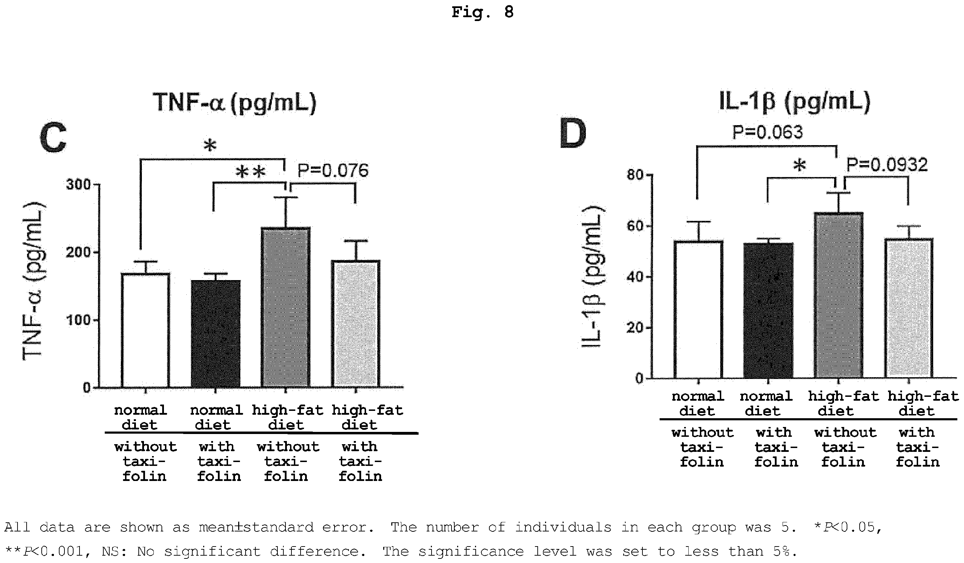

According to Fig. 8C, TNF-α concentration showed a tendency to decrease (P=0.076) in the high-fat diet + taxifolin group (with taxifolin) compared to the high-fat diet only group (without taxifolin).

According to Fig. 8D, IL-1β concentration showed a tendency to decrease (P=0.0932) in the high-fat diet + taxifolin group (with taxifolin) compared to the high-fat diet only group (without taxifolin).

### (x) Liver function test

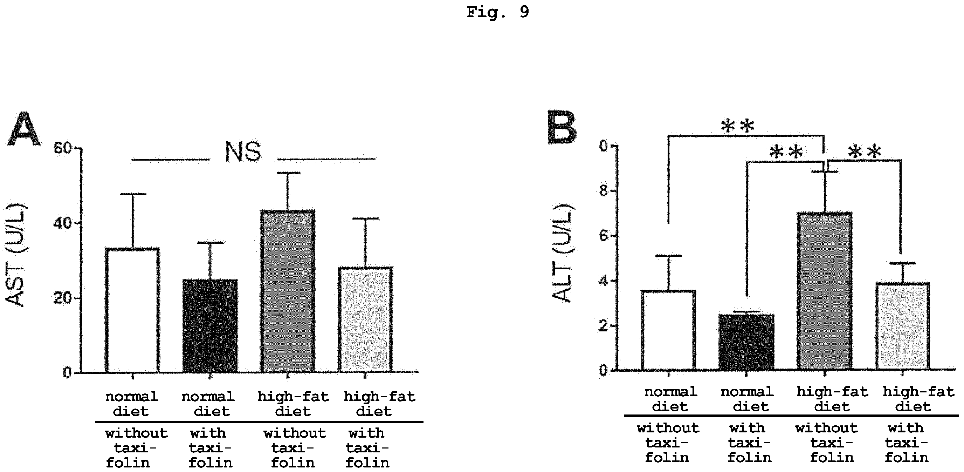

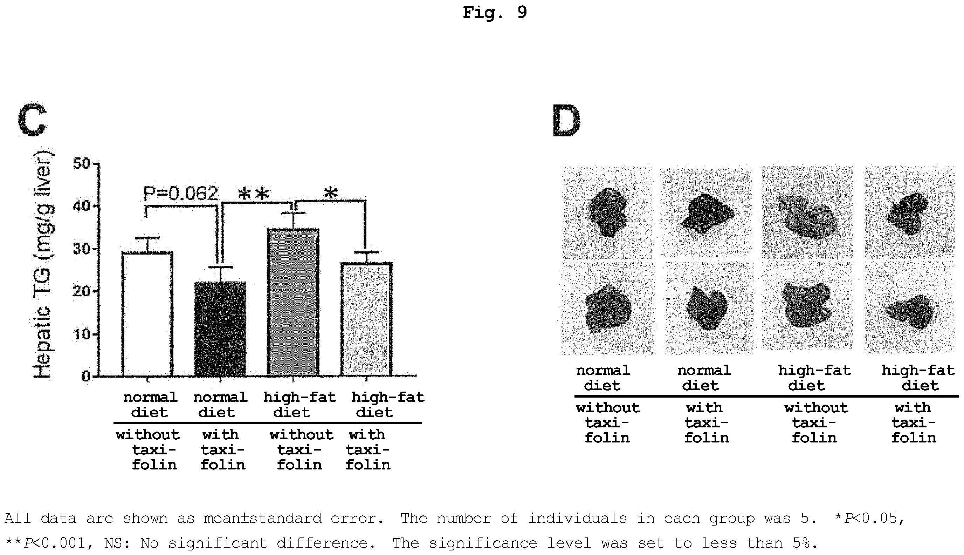

According to Fig. 9B, blood ALT levels were confirmed to be significantly decreased in the high-fat diet + taxifolin group (with taxifolin) compared to the high-fat diet only group (without taxifolin).

According to Fig. 9C, liver triglyceride concentration was confirmed to be significantly decreased in the high-fat diet + taxifolin group (with taxifolin) compared to the high-fat diet only group (without taxifolin).

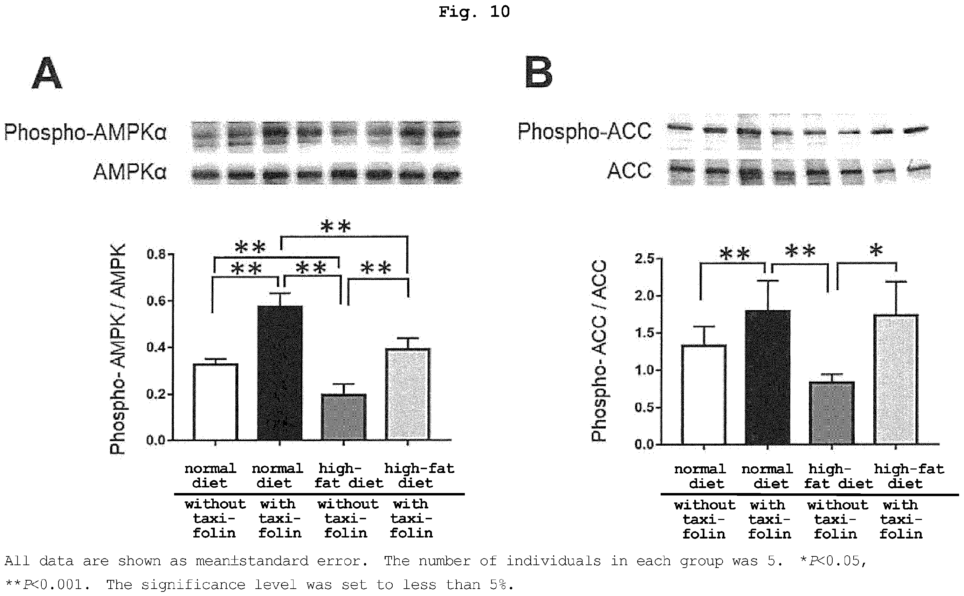

### (xi) Liver AMPK/acetyl coenzyme A carboxylase (ACC) activity According to Fig. 10A, AMPK phosphorylation was confirmed to be significantly increased in the high-fat diet + taxifolin group (with taxifolin) compared to the high-fat diet only group (without taxifolin), and was also confirmed to be significantly increased in the normal diet + taxifolin group (with taxifolin) compared to the normal diet only group (without taxifolin).

According to Fig. 10B, ACC phosphorylation was confirmed to be significantly increased in the high-fat diet + taxifolin group (with taxifolin) compared to the high-fat diet only group (without taxifolin), and was also confirmed to be significantly increased in the normal diet + taxifolin group (with taxifolin) compared to the normal diet only group (without taxifolin).

### (xii) Hepatic fatty acid synthesis/fatty acid oxidation

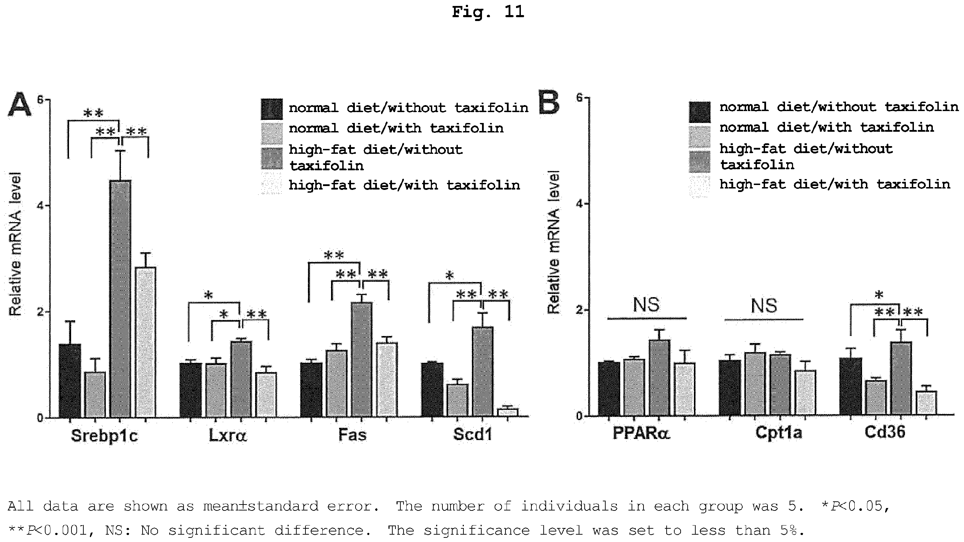

According to Fig. 11A, the expression of Srebplc, Lxrα, FAS, and Scd1 genes was confirmed to be significantly decreased in the high-fat diet + taxifolin group (high-fat diet/with taxifolin) compared to the high-fat diet only group (high-fat diet/without Taxifolin).

According to Fig. 11B, it was confirmed that the expression of Cd36 gene was significantly decreased in the high-fat diet + taxifolin group (high-fat diet/with taxifolin) compared to the high-fat diet only group (high-fat diet/without Taxifolin).

### (xiii) Oxidative stress and anti-oxidation effects

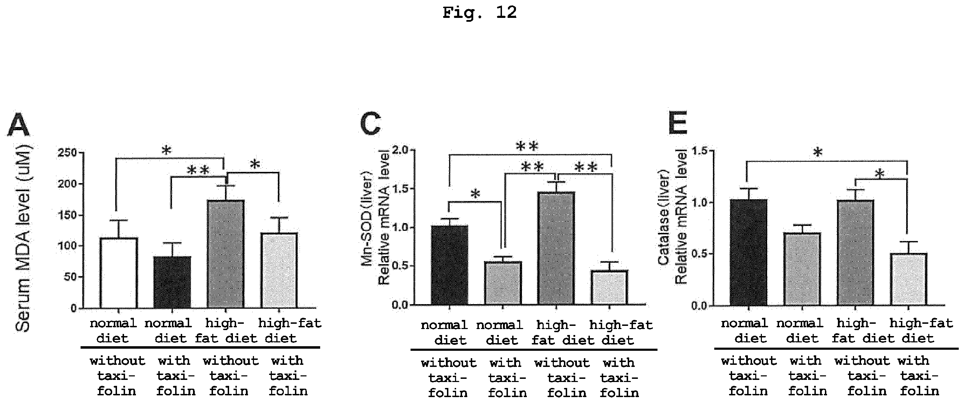

According to Fig. 12A, the concentration of malondialdehyde (MDA), a marker of lipid peroxidation in blood, was confirmed to be significantly decreased in the high-fat diet + taxifolin group (with taxifolin) compared to the high-fat diet only group (without taxifolin).

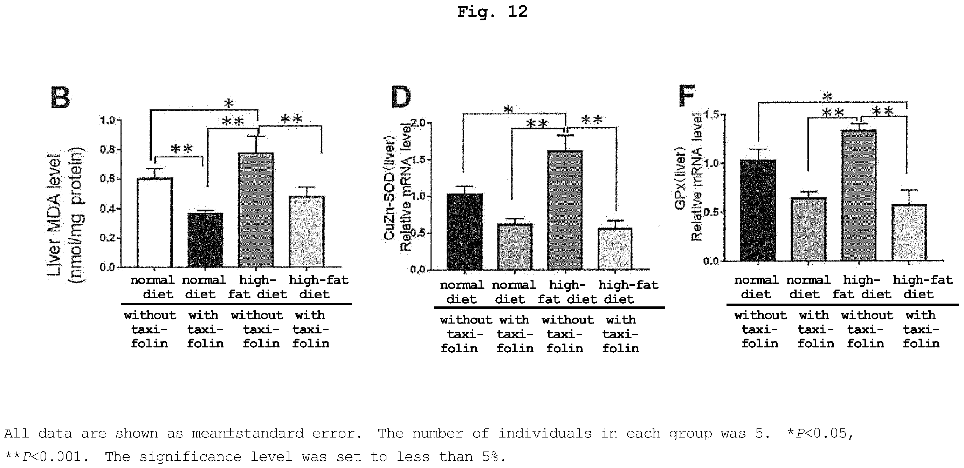

According to Fig. 12B, the concentration of MDA in the liver, a marker of hepatic lipid peroxidation, was confirmed to be significantly decreased in the high-fat diet + taxifolin group (with taxifolin) compared to the high-fat diet only group (without taxifolin), and was also confirmed to be significantly decreased in the normal diet + taxifolin group

(with taxifolin) compared to the normal diet only group (without taxifolin).

In addition, according to Fig. 12C, D, E, and F, gene expression of a group of anti-oxidation enzymes (Mn-SOD, CuZn-SOD, Catalase, GP-x) induced by oxidative stress was confirmed to be significantly decreased in the high-fat diet + taxifolin group (with taxifolin) compared to the high-fat diet only group (without taxifolin). Furthermore, according to Fig. 12C, the gene expression of Mn-SOD was confirmed to be significantly decreased even in the normal diet + taxifolin group (with taxifolin) compared to the normal diet only group (without taxifolin).

### (xiv) Liver tissue inflammation indicator gene expression

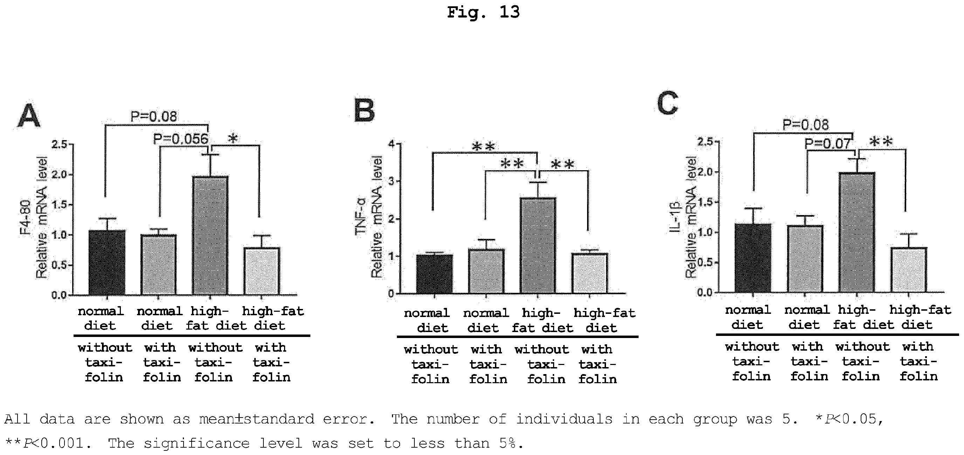

According to Fig. 13A, mature macrophage marker F4/80 gene expression was confirmed to be significantly decreased in the high-fat diet + taxifolin group (with taxifolin) compared to the high-fat diet only group (without taxifolin).

According to Fig. 13B and C, TNF-α and IL-1β gene expression was confirmed to be significantly decreased in the high-fat diet + taxifolin group (with taxifolin) compared to the high-fat diet only group (without taxifolin).

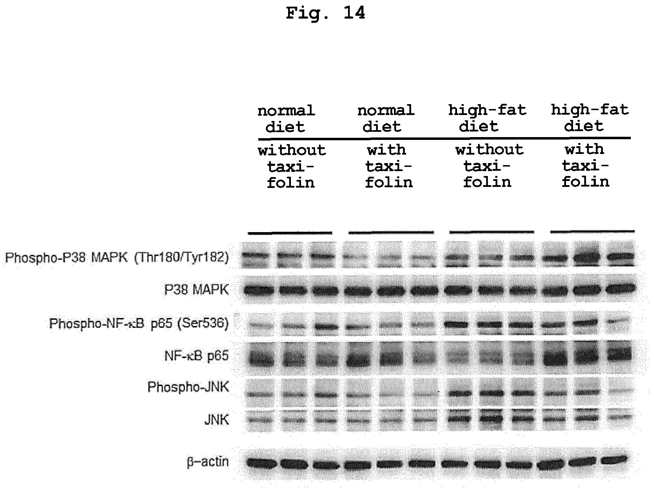

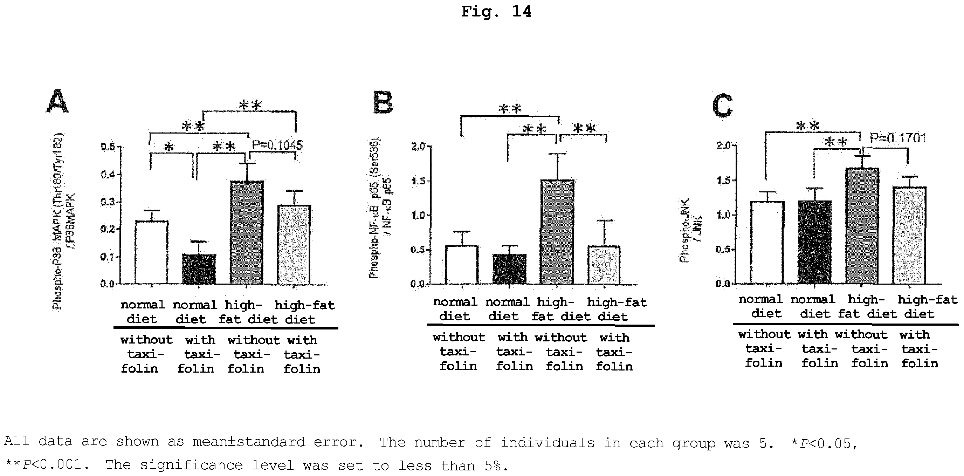

### (xv) Inflammation-induced signaling pathway activity According to Fig. 14A, P38 MAPK phosphorylation showed a decreasing trend in the high-fat diet + taxifolin group (with taxifolin) compared to the high-fat diet only group (without taxifolin) (P = 0.1045).

According to Fig. 14B, it was confirmed that NF-KB p65 phosphorylation was significantly decreased in the high-fat diet + taxifolin group (with taxifolin) compared to the high-fat diet only group (without taxifolin).

According to Fig. 14C, c-Jun amino-terminal kinase (JNK) phosphorylation showed a decreasing trend (P=0.1701) in the high-fat diet + taxifolin group (with taxifolin) compared to the high-fat diet only group (without taxifolin).

### (xvi) Liver fibrosis index analysis

### (xvi) Gene expression analysis

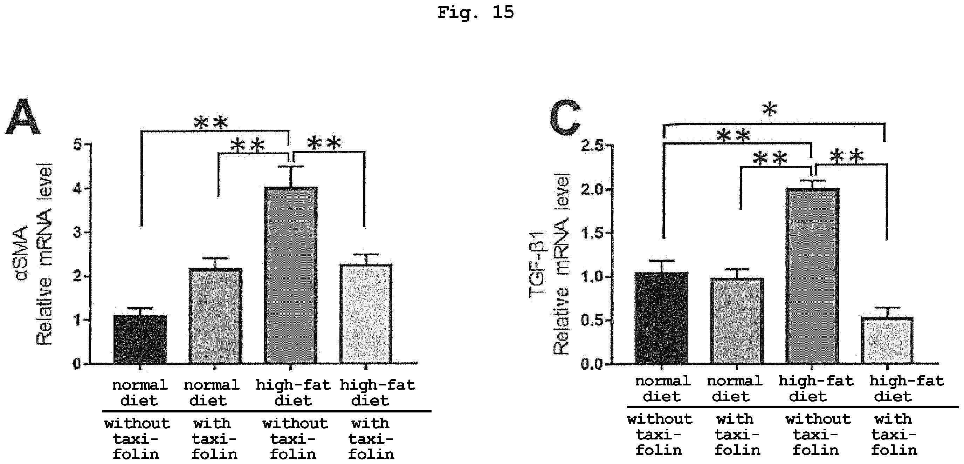

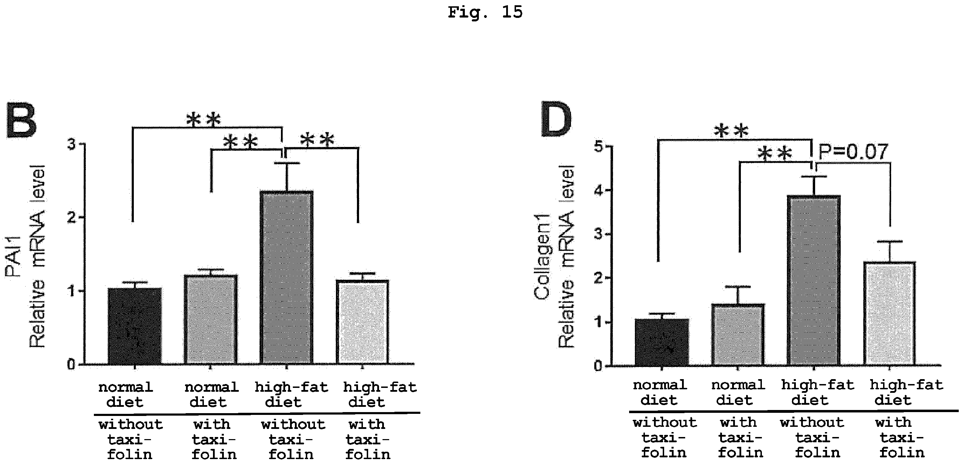

According to Fig. 15A-C, gene expression of fibrosis indicator groups (αSMA, PAI1, TGF-β1) was confirmed to be significantly decreased in the high-fat diet + taxifolin group (with taxifolin) compared to the high-fat diet only group (without taxifolin).

According to Fig. 15D, Collagen 1 was confirmed to show a decreasing trend (P=0.07) in the high-fat diet + taxifolin group (with taxifolin) compared to the high-fat diet only group (without taxifolin).

### (Protein content analysis)

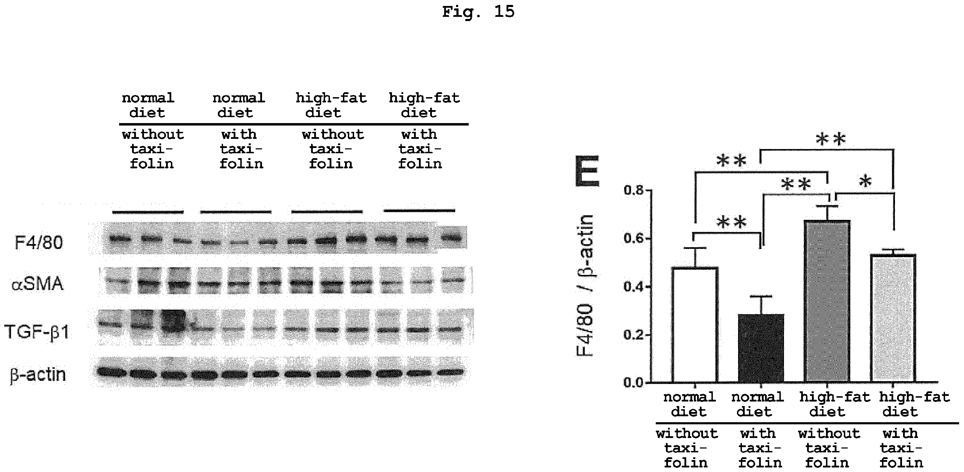

According to Fig. 15E, F4/80 was confirmed to be significantly decreased in the high-fat diet + taxifolin group (with taxifolin) compared to the high-fat diet only group (without taxifolin). It was also confirmed that the decrease was significant in the normal diet + taxifolin group (with taxifolin) compared to the normal diet only group (without taxifolin).

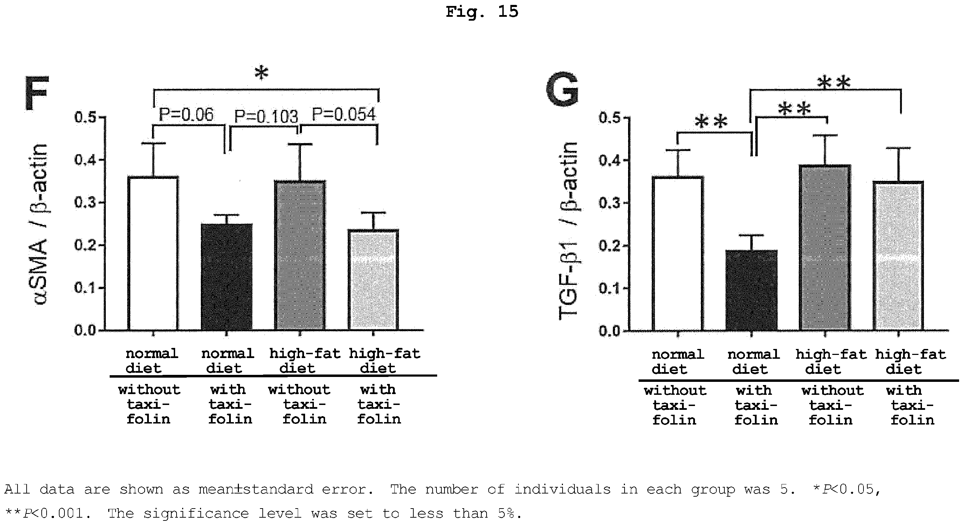

According to Fig. 15F, αSMA was confirmed to show a decreasing trend (P = 0.054) in the high-fat diet + taxifolin group (with taxifolin) compared to the high-fat diet only group (without taxifolin).

According to Fig. 15G, TGF-β1 was confirmed to be significantly decreased in the normal diet + taxifolin group (with taxifolin) compared to the normal diet only group (without taxifolin).

### (xvii) Histological observation

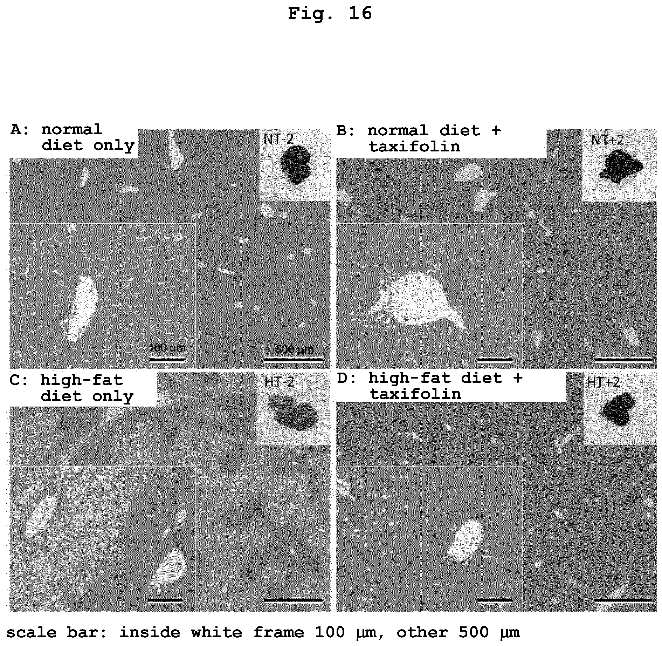

### (xvii) According to the Hematoxylin Eosin (HE) staining photograph in Fig. 16, significant fat accumulation in the high-fat diet-only group was confirmed. On the other hand, although fat accumulation was observed in the high-fat diet + taxifolin group, it was markedly reduced compared to the high-fat diet only group, confirming the suppression of fatty liver.

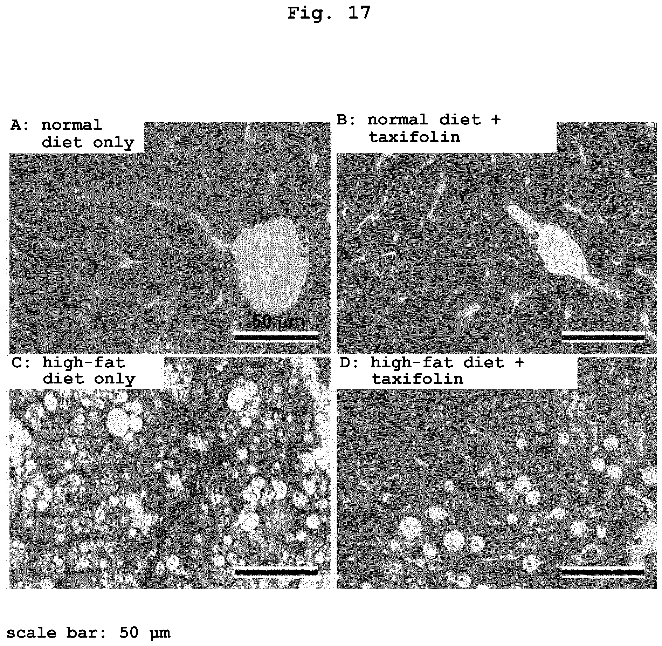

According to the Azan staining photograph in Fig. 17 (the blue fibrous area indicates collagen fibers), the amount of collagen stained blue was reduced in the high-fat diet + taxifolin group compared to the high-fat diet only group, confirming the suppression of fibrosis in liver tissue.

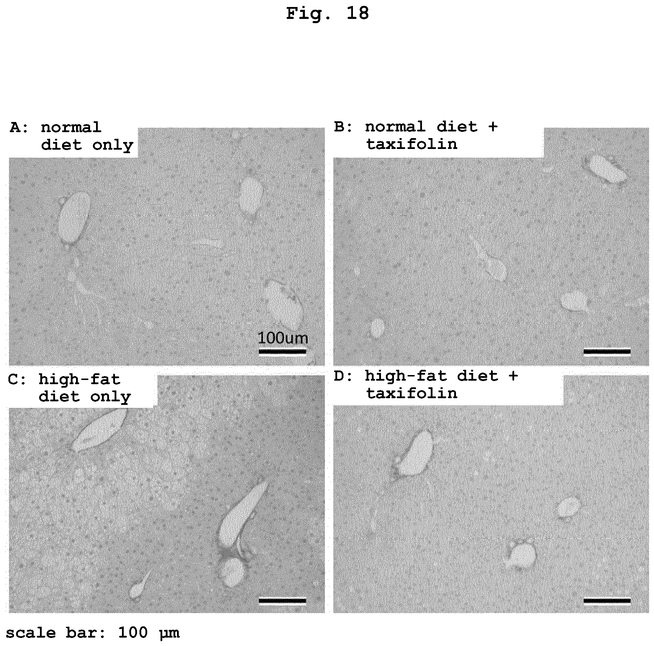

According to the photograph of Sirius Red staining in Fig. 18 (the red fibrillar area indicates collagen fibrils), the amount of collagen stained red in the high-fat diet + taxifolin group was decreased compared to the high-fat diet only group, and as in the Azan staining in Fig. 17, it was confirmed that the fibrosis of liver tissue was inhibited.

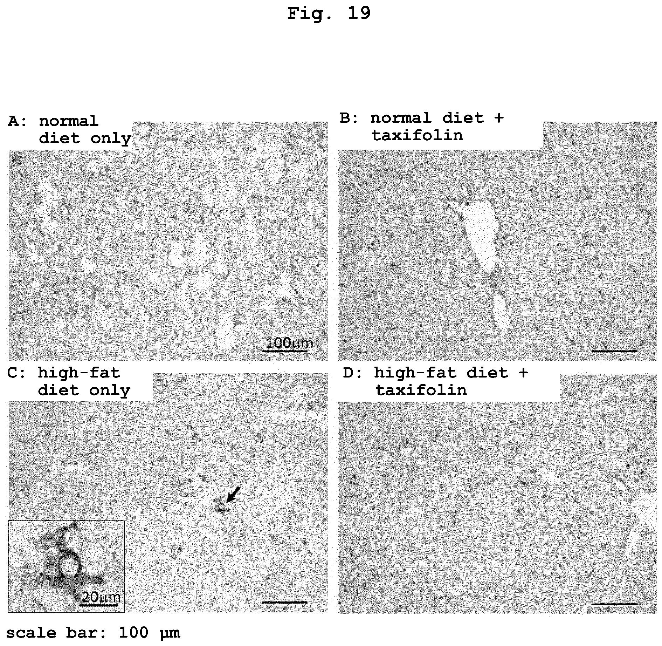

According to the immunohistochemical staining pictures using anti-F4/80 antibody in Fig. 19, hepatic crown-like structures (hCLS) (see the partially enlarged picture) tended to be observed in the high-fat diet-only group, but were hardly observed in the high-fat diet + taxifolin group, confirming the suppression of inflammation in liver tissue.

### Formulation Example 1 (production of tablet)

| | |
|---|---|
| 1) taxifolin | 50 g |
| 2) lactose | 50 g |
| 3) cornstarch | 15 g |
| 4) carboxymethylcellulose calcium | 44 g |
| 5) magnesium stearate | 1 g |
| total 1000 tablets | 160 g |

The total amount of 1), 2), 3) and 30 g of 4) are kneaded with water, vacuum dried, and sieved. The sieved powder is mixed with 14 g of 4) and 1 g of 5), and the mixture is punched by a tableting machine. In this manner, 1000 tablets containing 50 mg of taxifolin per tablet are obtained.

### Formulation Example 2 (production of capsule)

| | |
|---|---|
| 1) taxifolin | 50 mg |
| 2) fine powder cellulose | 10 mg |
| 3) lactose | 19 mg |
| 4) magnesium stearate | 1 mg |
| total | 80 mg |

| | |
|---|---|
| 1), 2), 3) and 4) are mixed and filled in a gelatin capsule. | |

### [Industrial Applicability]

According to the present invention, a superior liver fibrosis inhibiting agent characteristically containing taxifolin, which is a safe plant-derived component, or a salt thereof as an active ingredient can be provided. Using this, the progression of fibrosis in NASH can be suppressed, which in turn makes it possible to prevent aggravation of NASH. According to the present invention, moreover, a superior brown adipocyte activating agent containing taxifolin or a salt thereof as an active ingredient can be provided. Using this, it is possible to promote energy consumption, enhance the expression of UCP1, and effectively suppress the fibrosis of adipose tissue. As a result, metabolic syndrome and the like can be prevented or improved. Furthermore, according to the present invention, a medicament composition or food composition useful for the prophylaxis and/or treatment (or improvement) of liver fibrosis induced by various causes (hepatitis virus infection, NASH, autoimmune hepatitis, etc.), by administering a prophylactically and/or therapeutically effective amount of taxifolin or a salt thereof, which is a liver fibrosis inhibiting agent and/or a brown adipocyte activating agent, to a subject can be provided. Among others, according to the present invention, a novel and effective prophylactic and/or therapeutic agent for NASH accompanied by fibrosis, for which there was no effective prophylactic or treatment method, can be provided. It is also possible to potentiate the prophylactic and/or treatment effects of taxifolin or a salt thereof in the present invention by combining with dietary therapy, exercise therapy, drug therapy (combination therapy with other drugs), iron removal therapy, and/or surgical treatment (debulking operation, liver transplantation).

This application is based on a patent application No. 2019-117229 filed in Japan (filing date: June 25, 2019), the contents of which are incorporated in full herein.

## Claims

1. A liver fibrosis inhibiting agent comprising taxifolin as an active ingredient.

2. The liver fibrosis inhibiting agent according to claim 1, wherein the liver fibrosis is caused by hepatitis virus infection, nonalcoholic steatohepatitis, or autoimmune hepatitis.

3. The liver fibrosis inhibiting agent according to claim 1, wherein the liver fibrosis is caused by nonalcoholic steatohepatitis.

4. A brown adipocyte activating agent comprising taxifolin as an active ingredient.

5. The brown adipocyte activating agent according to claim 4 for suppressing liver fibrosis.

6. The brown adipocyte activating agent according to claim 5, wherein the liver fibrosis is caused by hepatitis virus infection, nonalcoholic steatohepatitis, or autoimmune hepatitis.

7. The brown adipocyte activating agent according to claim 5, wherein the liver fibrosis is caused by nonalcoholic steatohepatitis.

8. A prophylactic and/or therapeutic agent for nonalcoholic steatohepatitis accompanied by liver fibrosis, comprising the agent according to any one of claims 1 to 7 as an active ingredient.
